# EUROPEAN PATENT SPECIFICATION

(11) **EP 1 233 943 B1**
(45) Date of publication and mention of the grant of the patent: **29.06.2011**
(21) Application number: 00982228.9
(22) Date of filing: 22.11.2000
(51) Int. Cl.: C07C 403/06, A61K 31/404, A61P 43/00, C07D 403/14

(54) **IONIZABLE INDOLINONE DERIVATIVES AND THEIR USE AS PTK LIGANDS**
IONISIERBARE INDOLINON DERIVATE UND DEREN VERWENDUNG ALS PTK LIGANDEN
FORMULATIONS POUR AGENTS PHARMACEUTIQUES IONISABLES COMME ACIDES LIBRES OU BASES LIBRES

(30) Priority: 24.11.1999 US 167544 P
(43) Date of publication of application: 28.08.2002
(73) Proprietor: Sugen, Inc., New York, NY 10017 (US)
(72) Inventor: SHENOY, Narmada, Sunnyvale, CA 94087 (US); SORASUCHART, Waranush, Rajtavi Bangkok 10400 (TH); KOPARKAR, Arun, San José, CA 95120 (US)
(74) Representative: Ruddock, Keith Stephen
(86) International application number: PCT/US2000/032277
(87) International publication number: WO 2001/037820

(56) References cited:
- WO-A-00/08202
- WO-A-00/35908
- WO-A-00/38519
- WO-A-00/56709
- WO-A-98/07695
- WO-A-98/50356
- WO-A-99/61422
- US-A- 5 883 113
- US-A- 5 883 116
- US-A- 5 886 020

## Description

The present application claims priority to U.S. App. Ser. No. 60/167,544, filed November 24,1999.

### FIELD OF THE INVENTION

The instant invention provides formulations of 3-[2,4-dimethyl-5-(2-oxo-1,2-dihydro-indol-3-ylidenemethyl)-1H-pyrrol-3-yl]-propionic acid, which is ionizable as free acid or free base. Also provided are methods of making and using the formulations of the invention.

### BACKGROUND OF THE INVENTION.

The following description of the background of the invention is provided to aid in understanding the invention, but is not admitted to describe or constitute prior art to the invention. Various methods are available for administering therapeutic agents to a patient. Such methods include parenteral, oral, ocular, nasal, topical, and transmucosal administration. Variations of these different types of administrations exist. For example, parenteral administration includes intravenous, subcutaneous, intraperitoneal, intramuscular, intraosseous, and intramedullary injection. The chosen mode of administration should take into account the nature of the therapeutic compound and the illness being treated.

Certain potential pharmaceuticals are hydrophobic and typically have very low aqueous solubility and hence low oral bioavailability. Different techniques concerned with solubilizing hydrophobic compounds include those described by Praveen et al., U.S. Patent No. 5,314,685, and Fernandes et al., U.S. Patent No. 4,992,271

U.S. Patent No. 5,886,020 discloses 3-(4-dimethylaminobenzylidenyl)-2-indolinone and analogues thereof as organic molecules capable of modulating tyrosine kinases signal transduction in order to regulate, modulate and/or inhibit abnormal cell proliferation.

U.S. Patent No. 5883116 discloses 3-(2-alkoxybenzylidenyl)-2-indolinone and analogues thereof as organic molecules capable of modulating tyrosine kinase signal transduction in order to regulate, modulate and/or inhibit abnormal cell proliferation.

U.S. Patent No. 5883113 discloses 3-(4-Bromobenzylindenyl)-2-indolinone and analogues thereof as organic molecules capable of modulating tyrosine kinase signal transduction in order to regulate, modulate and/or inhibit abnormal cell proliferation.

WO 98/50356 discloses 2-indolidone derivatives and physiologically acceptable salts and prodrugs thereof which modulate the activity of protein kinases and therefore are expected to be useful in the prevention and treatment of protein kinase related cellular disorders such as cancer.

WO 98/07695 discloses indolidone combinatorial Libraries and related products and methods for the treatment of disease. Specifically, it relates to organic molecules capable of modulating, regulating and/or inhibiting protein kinase signal transduction. Such compounds are useful for the treatment of diseases related to unregulated protein kinase signal transduction, including cell proliferative diseases such as cancer, atherosclerosis, arthritis and restenosis and metabolic diseases such as diabetes. The document features indolinone compounds that potentially inhibit protein kinases and related products and methods. Inhibitors specific to the FLK protein kinase can be obtained by adding chemical substituents to the 3-[(indole-3-yl)methylene]-2-indolinone, in particular at the 1' position of the indole ring. Indolinone compounds that specifically inhibit the FLK and platelet derived growth factor protein kinases can harbor a tetrahydroindole or cyclopentano-b-pyrrol moiety.

WO 99/61422 discloses pyrrole substituted 2-indolinone compounds and physiologically acceptable salts and prodrugs thereof which modulate the activity of protein kinases and therefore are expected to be useful in the prevention and treatment of protein kinase related cellular disorders such as cancer.

WO 00/56709 discloses indolidone compounds as kinase inhibitors. Specifically, it relates to certain indolinone compounds, their method of synthesis, and a combinatorial library consisting of the disclosed indolinone compounds. It also relates to methods of modulating the function of protein kinases using indolinone compounds disclosed therein and methods of treating diseases by modulating the function of protein kinases and related signal transduction pathways.

WO 99/38519 relates to an anticancer composition. Therein, an anticancer pharmaceutical suppressive preparation is useful in treating cancer. The preparation bas an affinity to stop division and differentiation of cancerogenous cells. The preparation consists of animal extracts.

WO 00/35908 relates to 4-alkenyl (and alkynyl) oxindoles as inhibitors of cyclin-dependent kinases, in particular CDK2. The document discloses 4-alkenyl,- and 4-alkynyl oxindoles having specified formulas and the pharmaceutically acceptable salts thereof, The compounds inhibit cyclin-dependent kinases (CDKs), in particular CDK2 and are useful as anti-proliferative agents in the treatment or control of cell proliferative disorders, in particular breast and colon tumors.

WO 00/08202 relates to 3-methylidenyl-2-indolinone modulators of protein kinase. Disclosed are 3-methylidenyl-2-indolinone compounds and physiologically acceptable salts and prodrugs thereof which modulate the activity of protein kinases and therefore are expected to be useful in the prevention and treatment of protein kinase related cellular disorders such as cancer.

One measure of the potential usefulness of an oral formulation of a new pharmaceutical agent is the bioavailability observed after oral administration of the formulation. Various factors can affect the oral bioavailability of the drug. These factors include aqueous solubility, drug absorption throughout the gastrointestinal tract, drug stability in gastrointestinal tract, and first pass effect. Aqueous solubility is one of the most important factors. The oral bioavailability of an aqueous solution formulation of a drug is generally used as the standard or the ideal bioavailability against which other oral formulations are measured. Formulations of drugs that increase the relative bioavailability of the drug as compared to an aqueous solution are desirable, especially with hydrophobic compounds.

### SUMMARY OF THE INVENTION

The instant invention features formulations of 3-[2,4-dimethyl-5-(2-oxo-1,2-dihydro-indol-3-ylidenemethyl)-1H-pyrrol-3-yl]-propionic acid, which is ionizable as free acid or free base.

In one aspect, the invention features a formulation suitable for parenteral or oral administration, said formulation comprising an ionizable substituted indolinone, and a pharmaceutically acceptable carrier therefor.

The term "ionizable substituted indolinone" as used herein refers to 3-[2,4-dimethyl-5-(2 oxo-1,2-dihydro-indol-3-ylidenemethyl)-1H-pyrrol-3-yl]-propionic acid. Physiologically acceptable salts of the compound are also within the scope of this invention.

The chemical formulae referred to herein may exhibit the phenomena of tautomerism and structural isomerism. For example, the compounds described herein may adopt an E or a Z configuration about the double bond connecting the 2-indolinone moiety to the pyrrole moiety or they may be a mixture of E and Z. This invention encompasses any tautomeric or structural isomeric form and mixtures thereof which possess the ability to modulate receptor tyrosine kinase (RTK), cellular tyrosine kinase (CTK) and/or serine tyrosine kinase (STK) activity and is not limited to any one tautomeric or structural isomeric form.

A method for the synthesis of 3-[2,4-dimethyl-5-(2-oxo-1,2-dihydro-indol-3-ylidenemethyl)-1H-pyrrol-3-yl]propionic acid suitable for use as a "pyrrole substituted 2-indolinones" in this invention comprises reacting 2-oxindole with 3-(2-Carboxyethyl)-2,4-dimethyl-5-formylpyrrole in a solvent, preferably in the presence of a base.

The reaction may be carried out in the presence of a base. The base may be an organic or an inorganic base. If an organic base is used, preferably it is a nitrogen base. Examples of organic nitrogen bases include, but are not limited to, diisopropylamine, trimethylamine, triethylamine, aniline, pyridine, 1,8-diazabicyclo[5.4.1]undec-7-ene, pyrrolidine and piperidine.

Examples of inorganic bases are, without limitation, ammonia, alkali metal or alkaline earth hydroxides, phosphates, carbonates, bicarbonates, bisulfates and amides. The alkali metals include, lithium, sodium and potassium while the alkaline earths include calcium, magnesium and barium.

In a presently preferred aspect of this method, when the solvent is a protic solvent, such as water or alcohol, the base is an alkali metal or an alkaline earth inorganic base, preferably, a alkali metal or an alkaline earth hydroxide.

It will be clear to those skilled in the art, based both on known general principles of organic synthesis and on the disclosures herein which base would be most appropriate for the reaction contemplated.

The solvent in which the reaction is carried out may be a protic or an aprotic solvent, preferably it is a protic solvent. A "protic solvent" is a solvent which has hydrogen atom(s) covalently bonded to oxygen or nitrogen atoms which renders the hydrogen atoms appreciably acidic and thus capable of being "shared" with a solute through hydrogen bonding. Examples of protic solvents include, without limitation, water and alcohols.

An "aprotic solvent" may be polar or non-polar but, in either case, does not contain acidic hydrogens and therefore is not capable of hydrogen bonding with solutes. Examples, without limitation, of non-polar aprotic solvents, are pentane, hexane, benzene, toluene, methylene chloride and carbon tetrachloride. Examples of polar aprotic solvents are chloroform, tetrahydro- furan, dimethylsulfoxide and dimethylformamide.

In a presently preferred aspect of this method, the solvent is a protic solvent, preferably water or an alcohol such as ethanol.

The reaction is carried out at temperatures greater than room temperature. The temperature is generally from about 30° C to about 150° C, preferably about 80°C to about 100°. C, most preferable about 75° C to about 85° C, which is about the boiling point of ethanol. By "about" is meant that the temperature range is preferably within 10° C of the indicated temperature, more preferably within 5° C of the indicated temperature and, most preferably, within 2° C of the indicated temperature. Thus, for example, by "about 75° C" is meant 75° C± 10° C, preferably 75° C± 5° C and most preferably, 75° C ± 2° C.

The ionizable substituted indolinone is 3-[2,4-dimethyl-5-(2-oxo-1,2-dihydro-indol-3-ylidenemethyl)-1H-pyrrol-3-yl]-propionic acid (compound IV), pharmaceutically active salts and derivatives thereof (cf. also Table 1).

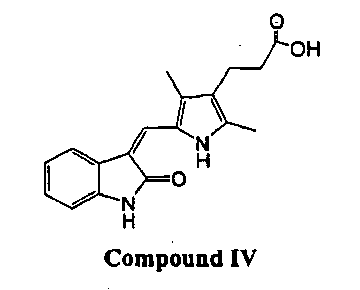

**TABLE 1**

| Example | Structure |
|---|---|
| 1 | 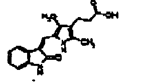 |
| 2 | 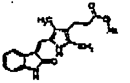 |

As utilized herein, the term "pharmaceutically acceptable salt" includes formulations of a compound that do not abrogate the biological activity and properties of the compound. Pharmaceutically acceptable salts can be obtained by reacting a pyrrole substituted 2-indolinone contemplated for use in the invention with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like, or with inorganic or organic bases such as sodium hydroxide, potassium hydroxide, ammonium hydroxide, calcium hydroxide, choline, n-methyl glucamine, diethylamine, procaine and the like. The pharmaceutically acceptable salts which the compounds of this invention may form include the negatively or the positively charged species. Examples of salts in which the compound forms the positively charged moiety include, without limitation, quaternary ammonium (defined elsewhere herein), salts such as the hydrochloride, sulfate, carbonate, lactate, tartrate, maleate, succinate wherein the nitrogen atom of the quaternary ammonium group is a nitrogen of the selected compound of this invention which has reacted with the appropriate acid. Salts in which a compound of this invention forms the negatively charged species include, without limitation, the sodium potassium, calcium and magnesium salts formed by the reaction of a carboxylic acid group in the compound with an appropriate base (e.g. sodium hydroxide (NaOH), potassium hydroxide (KOH), calcium hydroxide (Ca(OH)2), etc.). As utilized herein, "quaternary ammonium" includes a quaternized nitrogen (e.g., -NRR'R", where each of R,R' and R" is independently selected from H, aryl, alkyl, and the like), a quaternized nitrogen containing heterocyclic aryl, and the like.

In one aspect of the invention formulation, a therapeutically effective amount of the ionizable substituted indolinone is utilized in the invention formulation.

As used herein, a "pharmaceutically acceptable carrier" refers to a carrier or diluent that does not abrogate the biological activity and/or properties of the administered compound while facilitating administration by, for example, stabilizing or solubilizing the compound. Suitable pharmaceutically acceptable carriers include, without limitation, one or more polyoxyhydrocarbyl compounds, one or more buffers, one or more pharmaceutically acceptable surfactant, one or more pharmaceutically acceptable preservatives, one or more antioxidants, one or more pharmaceutically acceptable alcohols, one or more pharmaceutically acceptable aqueous solutions, one or more pharmaceutically acceptable oils, liposomes, one or more polyglycolized lipids, one or more pharmaceutically acceptable granulating agents, one or more pharmaceutically acceptable diluents, one or more pharmaceutically acceptable binders, one or more pharmaceutically acceptable disintegrants, one or more pharmaceutically acceptable lubricants, one or more pharmaceutically acceptable flow enhancers, one or more pharmaceutically acceptable suspending agents, and suitable combinations of any two or more thereof.

The term "pharmaceutically acceptable" or "pharmaceutical" as used herein refers to solutions or components of the formulation that do not prevent the therapeutic compound from exerting a therapeutic effect and do not cause unacceptable adverse side effects. Examples of pharmaceutically acceptable reagents are provided in The United Stares Pharmacopeia The National Formulary, United States Pharmacopeial Convention, Inc., Rockville, MD 1990 and FDA Inactive Ingredient Guide 1990, 1996 issued by the Division of Drug Information Resources. Unacceptable side effects vary for different diseases. Generally, the more severe the disease the more toxic effects which will be tolerated. Unacceptable side effects for different diseases are known in the art.

Although all pennutations of specific components within the invention formulations are contemplated to be within the scope of the present invention, the following combinations of one or more specific pharmaceutically acceptable carrier) and specific ionizable substituted indolinones are preferred in one aspect of the invention.

In one aspect of the invention formulation, the formulation is suitable for parenteral adminstration. The ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

In another aspect of the invention formulation suitable for parenteral or oral administration, the pharmaceutically acceptable carrier comprises one or more polyoxyhydrocarbyl compounds. The ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

In another aspect of the invention, the formulation includes permeability and penetrating enhancers. These include ionic compounds (e.g., 3,5-diidosalicylate sodium) dimethylsulfoxide and related compounds (e-g., decylmethyl sulfoxide) azone, and related compounds (e.g., N-alkyl-dihydro-1,4-oxazepine-5,7-diones), solvents, and related compounds (e.g., ethanol, dimethyl acetamide, dimethylformamide) fatty alcohols, fatty acids and enzymes (e.g.; acid phosphatase and papin). These are other examples of permeability and pentration enhancers can be found in Pharmaceutical Skin Penetration. Enhancement, K.A. Walters and J. Hadgraft, Eds. (Dekker, New York, 1993).

The term "polyoxyhydrocarbyl compound" as used herein includes without limitation water soluble carbohydrates (such as glucose, sucrose, maltotriose, and the like); water soluble carbohydrate derivatives (such as gluconic acid and mannitol and oligosaccharides, and the like); water soluble polypeptides; water soluble polymers (such as polyvinylpyrrolidone, poly(vinyl alcohol), and in particular, polyethers such as other polyoxyalkylenes including poly(ethylene glycol) and the like); water soluble mixed oxyalkylene polymers; and the polymeric forms of ethylene glycol; and the like; and suitable combinations of two or more thereof. Although polyoxyhydrocarbyl compounds preferably contain more than one carbon, oxygen, and hydrogen atom, some molecules such as poly(ethyleneimine) are also included.

A particularly preferred class of solubilizing polyoxyhydrocarbyl compounds comprises poly(ethylene glycol) (PEG) and PEG derivatives, such as PEG monomethyl ether. Other suitable PEG derivatives include PEG-silicon deprived ethers. Many of these polymers are commercially available in a variety of molecular weights. Others may be conveniently prepared from commercially available materials, such as by coupling of an amino-PEG moiety to a haloalkyl silyl or silane moiety.

Suitable PEGs may vary in molecular weight from about 200 g/mol to about 20,000 g/mol or more, more preferably 200 g/mol to 5,000 g/mol, even more preferably 250 g/mol to 1,000 g/mol, and most preferably 250 g/mol to 500 g/mol. The choice of a particular molecular weight may depend on the particular ionizable substituted indolinone chosen and its molecular weight and degree of hydrophobicity, as well as the particular application for which the formulation is to be used.

Thus, the one or more polyoxyhydrocarbyl compounds can be selected from the group consisting of polyethylene glycol 300, polyethylene glycol 400, propylene glycol, and glycerin, and the like, and suitable combinations of two or more hereof. Preferably, each of the one or more polyoxyhydrocarbyl compounds is polyethylene glycol 300.

In another aspect of the invention formulation suitable for parenteral or oral administration, the ionizable substituted indolinone is solubilized by combining it with a molar equivalent of a base solution or an acid solution, wherein the ionizable substituted indolinone is 3-[2,4-dimethyl-5-(2-oxo-1,2-dihydro-indol-3-ylidenemethyl)-1H-pyrro-3-yl]-propionic acid (compound IV), and pharmaceutically active salts and derivatives thereof.

The term "solubilized" as used herein refers to dissolving of a substance in a fluid and/or adsorption of fluid molecules on the surface of the substance to assist in such dissolving. In one aspect, "solubilized" refers to hydration of a substance in water.

The term "molar equivalent" as used herein refers to equal or similar molar amounts of a test substance as compared to a reference substance.

The term "base solution" as used herein refers to a basic solution, typically one which has a pH higher than 7 and is capable of reacting with an acidic solution. Preferably the base in the base solution is selected from the group consisting of sodium hydroxide, ammonium hydroxide, triethylamine, ethylenediamine, N-methyl-D-glucamine, choline, triethanolamine, and the like, and suitable combinations of two or more hereof.

The term "acid solution" as used herein refers to an acidic solution, typically one which has a pH lower than 7 and is capable of reacting with a basic solution. Preferably the acid in the acid solution is selected from the group consisting of hydrochloric acid, sulfuric acid, formic acid, lactic acid, malic acid, succinic acid, acetic acid, methane sulfonic acid, benzene sulfonic acid, phosphoric acid, and the like, and suitable combinations of two or more hereof.

In another aspect of the invention formulation suitable for parenteral or oral administration, the pharmaceutically acceptable carrier further comprises one or more buffers, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

The term "buffer" as used herein refers to a substance, preferably in a solution, that resists a change of quality. Preferably a buffer is a solution that resists a change to a pH, such as a substance in a solution capable of neutralizing both acids and bases and therefore maintaining an original acidity or basicity of a solution. Suitable buffers include acetate, citrate, phosphate buffer, ascorbate, hydrochloric acid buffer, Tris-HCl buffer, sodium phosphate, sodium carbonate, sodium hydroxide, glutamate, glycine, Tris base buffers, and the like, and suitable combinations of two or more hereof. Most preferably, the buffer is sodium phosphate buffer.

In one embodiment, the buffer pH is three pH units higher than the pka of the ionizable substituted indolinone, or three pH units lower than the pkb of the ionizable substituted indolinone. Preferably, the buffer has a molarity (i.e., molar concentration, measured in moles per liter (M)) between 0.01 M and 0.1 M.

The term "pka" as used herein refers to the negative logarithm of the acidity constant, the acidity constant being the product of the concentration of the hydronium ion and the concentration of the conjugated base, divided by the concentration of the acid (the acidity constant is also sometimes referred to as the equilibrium constant).

The term "pkb" as used herein refers to the negative logarithm of the basicity constant, the basicity constant being the product of the concentration of the hydroxyl ion and the concentration of the conjugated acid, divided by the concentration of the base (the basicity constant is also sometimes referred to as the equilibrium constant).

In another aspect of the invention formulation suitable for parenteral or oral administration, the pharmaceutically acceptable carrier further comprises one or more pharmaceutically acceptable surfactants, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

The term "pharmaceutically acceptable surfactant" as used herein with respect to both oral and parenteral formulations refers to a compound that can solubilize hydrophobic compounds into aqueous solutions. Suitable surfactants include non-ionic surfactants, anionic surfactants, and the like, and suitable combinations of two or more thereof.

Preferably for parenteral formulations, the surfactant is a non-ionic surfactant. Examples of pharmaceutically acceptable non-ionic surfactants include but are not limited to polyoxyethylene sorbitan fatty acid esters (e.g., POLYSORBATE 80®, and the like), glyceryl monooleate, sorbitan monooleate, lecithin, polyvinyl alcohol, ethylene oxide copolymers (such as PLURONIC™ (a polyether), TETRONIC™ (BASF), and the like), polyol moieties, sorbitan esters, and the like, and suitable combinations of two or more hereof. Preferably ethoxylated castor oils, such as CREMOPHOR EL^{®}, are used for the formulation of hydrophobic pharmaceutical agents, such as the ionizable substituted indolinones contemplated for use in the present invention. The term "ethoxylated castor oil" as used herein refers to castor oil that is modified with at least one oxygen containing moiety. In particular the term refers to castor oil comprising at least one ethoxyl moiety.

Further, the term "pharmaceutically acceptable surfactant", as used herein in reference to oral formulations, includes pharmaceutically acceptable non-ionic surfactants such as copolymers of ethylene glycol nd propylene glycol (for example, polyoxyethylenepolypropylene glycols (such as POLOXAMER® 68 (BASF Corp.)) or a mono fatty acid ester of polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), polyoxyethylene (20) sorbitan monolaurate (TWEEN® 20), and the like); polyoxyethylene castor oil derivatives (such as polyoxyethyleneglycerol-triricinoleate, polyoxyl 35 castor oil (CREMOPHOR® EL, BASF Corp.), and the like); polyoxyethyleneglycerol oxystearate (such as CREMOPHOR® RH 40 (polyethyleneglycol 40 hydrogenated castor oil), CREMOPHOR® RH 60 (polyethyleneglycol 60 hydrogenated castor oil), BASF Corp.), and the like); and the like); pharmaceutically acceptable anionic surfactants, e.g., sodiumlauryl sulfate (SLS); and the like; and suitable combinations of two or more hereof.

In a further aspect of the invention formulation suitable for parenteral or oral administration, the pharmaceutically acceptable carrier further comprises one or more pharmaceutically acceptable preservatives, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

Preferably, each of the one or more pharmaceutically acceptable preservatives is selected from the group consisting of benzyl alcohol, methyl paraben, ethyl paraben, propyl paraban, phenol, and the like, and suitable combinations of two or more hereof. A most preferred preservative is benzyl alcohol.

In yet another aspect of the invention formulation suitable for parenteral or oral administration, the pharmaceutically acceptable carrier further comprises one or more antioxidants, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

The term "antioxidant" as used herein refers to a substance that inhibits oxidation or reactions promoted by, for example, oxygen or peroxides. Suitable antioxidants include sodium meta-bisulfite, EDTA, sodium ascorbate, ascorbic acid, ascorbic acid palmitate, benzyl alcohol, alpha-tocopherol and the like, and suitable combinations of two or more hereof. Preferably the antioxidant is alpha-tocopherol.

In yet a further aspect of the invention formulation suitable for parenteral or oral administration, the pharmaceutically acceptable carrier further comprises one or more pharmaceutically acceptable alcohols, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

The term "pharmaceutically acceptable alcohol" as used herein refers to alcohols which are liquids at about room temperature (approximately 20°C). These include propylene glycol, ethanol, 2-(2-ethoxyethoxy)ethanol (TRANSCUTOL®, Gattefosse, (Westwood, NJ)), benzyl alcohol, glycerol, and the like, and suitable combinations of two or more hereof. Preferably, each of the one or more pharmaceutically acceptable alcohols is independently selected from the group consisting of ethanol, benzyl alcohol, propylene glycol, 2-(2-ethoxyethoxy)ethanol, and glycerol. Most preferably, each of the pharmaceutically acceptable alcohols is ethanol or polyethylene glycol.

The formulation including one or more pharmaceutically acceptable alcohols should be dissolved in a sufficient amount of a pharmaceutically acceptable aqueous solution prior to patient administration to avoid toxic effects due to the alcohol content. The added amount of a pharmaceutically acceptable aqueous solution should be sufficient to avoid hemolysis. Examples of suitable pharmaceutically acceptable aqueous solutions such as WFI (water for injection) and solutions containing isotonic saline are known in the art. Pharmaceutically acceptable aqueous solutions include 0.45% N saline, WFI (water for injection), D5W (5% dextrose in water), D5W 0.45% N saline, and the like, and suitable combinations of two or more hereof.

In a separate aspect of the invention is a formulation suitable for parenteral (especially subcutaneous or intramuscular) or oral administration, the pharmaceutically acceptable carrier further comprises one or more pharmaceutically acceptable oils, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

Suitable pharmaceutically acceptable oils include mineral oils, vegetable oils (e.g., safflower oil, peanut oil, olive oil, sesame oil, coconut oil, and the like, and suitable combinations of two or more hereof), fractionated coconut oil, propyleneglycol monolaurate, and mixed triglycerides with caprylic acid and capric acid, and the like, and suitable combinations of two or more hereof. In a preferred embodiment, the oil is Miglyol 812 (a mixture of triestyer of glycerin and caprylic and capric acids-capric/caprylic triglyceride). In another preferred embodiment, the oil is sesame oil.

In a particular aspect, the invention features a formulation suitable for parenteral administration, the formulation comprising: (a) 3-[2,4-dimethyl-5-(2-oxo-1,2-dihydro-indol-3-ylidenemethyl)-1H-pyrrol-3-yl]-propionic acid; (b) polyethylene glycol 300; and (c) sodium phosphate buffer. In preferred embodiments, the parenteral formulation also contains benzyl alcohol, and 3-[2,4-dimethyl-5-(2-oxo-1,2-dihydro-indol-3-ylidenemethyl)-1H-pyrrol-3-yl]-propionic acid is solubilized with sodium hydroxide.

In a further aspect of the invention formulation, the formulation comprising an ionizable substituted indolinone and a pharmaceutically acceptable carrier is suitable for oral administration, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivative thereof.

In one aspect of the invention formulation suitable for oral administration, the pharmaceutically acceptable carrier comprises one or more polyoxyhydrocarbyl compounds, wherein the ionizable substituted indolinone is 3-[2,4-dimethyl-5-(2-oxo-1,2-dihydro-indol-3-yildenemethyl)-1H-pyrrol-3-yl]-propionic acid (compound IV), and pharmaceutically active salts and derivatives thereof.

In preferred embodiments of this aspect of the invention formulation suitable for oral administration, the one or more polyoxyhydrocarbyl compounds are independently selected from the group consisting of: water soluble carbohydrates, water soluble carbohydrate derivatives, polypeptides, water soluble polymers, water soluble mixed oxyalkylene polymers, and the polymeric form of ethylene glycol.. Preferably, the one or more polyoxyhydrocarbyl compounds are poly(ethylene glycol) (PEG) or PEG derivatives. More preferably, PEG may vary in molecular weight from about 200 daltons to about 20,000 daltons.

In another aspect of the invention formulation suitable for oral administration, the pharmaceutically acceptable carrier comprises one or more polyglycolized lipids, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts derivatives thereof.

The term "polyglycolized lipids" as used herein refers to mixtures of monoglycerides, diglycerides, or triglycerides and polyethyleneglycol monoesters and diesters formed by the partial alcoholysis of vegetable oil using PEG of 200 g/mol to 2,000 g/mol or by the esterification of fatty acids using PEG 200 g/mol to 2,000 g/mol and glycerols. Preferably these include GELUCIRE® 35/10, GELUCIRE® 44/14, GELUCIRE® 46/07, GELUCIRE® 50/13, GELUCIRE® 53/10, and LABRASOL®.

In another aspect of the invention formulation suitable for oral administration, the pharmaceutically acceptable carrier comprises one or more pharmaceutically acceptable surfactants, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

In an additional aspect of the invention formulation suitable for oral administration, the pharmaceutically acceptable carrier comprises one or more pharmaceutically acceptable granulating agents, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof. Suitable granulating agents include without limitation silicon dioxide, microcrystalline cellulose, starch, calcium carbonate, pectin, crospovidone, polyplasdone; and the like, and suitable combinations of two or more thereof.

In an additional aspect of the invention formulation suitable for oral administration, the pharmaceutically acceptable carrier comprises suitable combinations of two or more of the members of the group comprising one or more polyoxyhydrocarbyl compounds, one or more polyglycolized lipids, one or more surfactants, and one or more granulating agents. Preferably, the pharmaceutically acceptable carrier comprises one or more polyoxyhydrocarbyl compounds, one or more polyglycolized lipids, and one or more surfactants. These oral formulations have advantageous solubility characteristics and oral bioavailability, and allow for the oral administration of the ionizable substituted indolinones for testing.

In an additional aspect, the invention provides pharmaceutically acceptable compositions containing an ionizable substituted indolinone. Preferred pharmaceutically acceptable compositions of the present invention are selected from the group comprising the invention formulation suitable for oral administration, a hard gelatin capsule filled with the invention formulation suitable for oral administration, a soft gelatin capsule filled with the invention formulation suitable for oral administration, and a hard gelatin capsule filled with the invention formulation suitable for oral administration admixed with a granulating agent to form a dry solid composition. In preferred embodiments, a solution comprising the invention formulation suitable for oral administration is encapsulated in a soft elastic gelatin capsule (SEC) or a hard gelatin capsule.

A solid composition of the formulation can be prepared by mixing the invention formulation in a liquefied state with a pharmaceutically acceptable granulating agent or a mixture of pharmaceutically acceptable granulating agents.

In an additional aspect of the invention formulation suitable for oral administration, the formulation is solid and the pharmaceutically acceptable carrier comprises one or more pharmaceutically acceptable diluents, one or more pharmaceutically acceptable binders, one or more pharmaceutically acceptable disintegrants, one or more pharmaceutically acceptable surfactants, one or more pharmaceutically acceptable lubricants and one or more pharmaceutically acceptable flow enhancers, wherein the ionizable substituted indolinone is 3-[2,4-dimethyl-5-(2-oxo-1,2-dihydro-indol-3-ylidenemethyl)-1H-pyrrol-yl]-propionic acid (compound IV), and pharmaceutically active salts and derivatives thereof.

Suitable pharmaceutically acceptable diluents include without limitation pregelatinized starch, lactose, monohydrate or lactose anhydrous, mannitol, microcrystalline cellulose, and the like, and suitable combinations of two or more thereof. Suitable pharmaceutically acceptable binders include without limitation polyvinylpyrrolidone, hydroxylpropyl cellulose, hydroxypropylmethylcellulose, starch, and the like, and suitable combinations of two or more thereof. Suitable pharmaceutically acceptable disintegrants include without limitation sodium starch glycollate, crosscarmellose, crospovidone, sodium carboxymethylcellulose, calcium carboxymethylcellulose, starch and the like, and suitable combinations of two or more thereof. Suitable pharmaceutically acceptable surfactants are as described herein, and further include without limitation sodium lauryl sulfate, cetylpyridinium chloride, polysorbate 80, polyoxyethylene stearates, and the like, and suitable combinations of two or more thereof. Suitable pharmaceutically acceptable lubricants include without limitation magnesium stearate, stearic acid, sodium stearyl fumarate, PEG (3,000-10,000), glyceryl behenate and the like, and suitable combinations of any two or more thereof. Suitable pharmaceutically acceptable flow enhancers include without limitation colloidal silicon dioxide, talc, and the like, and suitable combinations of any two or more thereof.

In another aspect of the invention formulation suitable for oral administration, the formulation is a solution and the pharmaceutically acceptable carrier comprises one or more polyoxyhydrocarbyl compounds, one or more surfactants and one or more buffers, wherein the ionizable substituted indolinone is 3-[2,4-dimethyl-5-(2-oxo-1,2-dihydro-indol-3-ylidenthyl)-1H-pyrrol-3-yl]-propionic acid, and pharmaceutically active salts and derivatives thereof.

In a further aspect of the invention formulation suitable for oral administration, the formulation is a suspension and the pharmaceutically acceptable carrier comprises one or more pharmaceutically acceptable suspending agents, one or more pharmaceutically acceptable salt solutions and one or more pharmaceutically acceptable surfactants, wherein the ionizable substituted indolinone is 3-[2,4-dimethyl-5-(2-oxo-1,2-dihydro-indol-3-ylidenemethyl)-1H-pyrrol-3-yl]-propionic acid, and pharmaceutically active salts and derivatives thereof.

Suitable pharmaceutically acceptable suspending agents include without limitation povidone, carboxylmethycellulose (CMC) and hydroxypropylmethyl cellulose (HPMC).

In yet another aspect of the invention formulation suitable for oral administration, the formulation is a solution and the pharmaceutically acceptable carrier comprises one or more pharmaceutically acceptable oils, and one or more pharmaceutically acceptable surfactants, wherein the ionizable substituted indolinone is 3-[2,4-dimethyl-5-(2-oxo-1,2-dihydro-indol-3-ylidenemethyl)-1H-pyrrol-3-yl]-propionic acid, and pharmaceutically active salts and derivatives thereof. In other preferred embodiments, each of the one or more pharmaceutically acceptable oils is a sesame oil, and each of the one or more pharmaceutically acceptable surfactants is an ethylene oxide copolymer (e.g., PLURONIC™ F68, and the like, as described above).

In yet a further aspect of the invention formulation suitable for oral administration, the pharmaceutically acceptable carrier comprises one or more pharmaceutically acceptable surfactants and one or more pharmaceutically acceptable oils, wherein the ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof. In a preferred embodiment, the pharmaceutically acceptable surfactant comprises ethylene oxide copolymer (e.g., PLURONIC™ F68, and the like) and the pharmaceutically acceptable oil comprises sesame oil.

In an additional aspect, the invention features a method of preparing a formulation for parenteral or oral administration comprising adding to a salt solution, formed in situ by admixing a molar equivalent of a base solution or an acid solution with an ionizable substituted indolinone, one or more polyoxyhydrocarbyl compounds and/or one or more buffers. In a preferred embodiment, both the one or more polyoxyhydrocarbyl compounds and the one or more buffers are added to the salt solution, wherein the ionizable substituted indolinone is 3-[2,4-dimethyl-5-(2-oxo-1,2-dihydro-indol-3-ylidenemetyl)-1H-pyrrol-3-yl]-propionic acid, and pharmaceutically active salts and derivatives thereof.

In a preferred embodiment of the method of preparing a formulation, the acid solution is selected from the group consisting of hydrochloric acid, sulfuric acid, formic acid, lactic acid, malic acid, and the like, and suitable combinations of two or more hereof, and the base solution is selected from the group consisting of sodium hydroxide, ammonium hydroxide, meglumine, triethylamine, triethanolamine, and the like, and suitable combinations of two or more hereof. Preferably, the base solution is sodium hydroxide.

In another preferred embodiments of the method of preparing a formulation, the one or more polyoxyhydrocarbyl compounds is selected from the group consisting of polyethylene glycol 300, polyethylene glycol 400, propyleneglycol, glycerin, and the like, and suitable combinations of two or more thereof, although polyoxyhydrocarbyl compounds listed previously can also be used in some cases. Preferably, the one or more polyoxyhydrocarbyl compounds is polyethylene glycol 300.

In a further preferred embodiment of the method of preparing a formulation, the bluffer pH is three pH units higher than the pka of the ionizable substituted indolinone or three pH units tower than the pkb of the ionizable substituted indolinone, and has a polarity between 0.01 M and 0,1 M. Preferably, the buffer is selected from the group consisting of acetate, citrate, phosphoric acid buffer, ascorbate, hydrochloric acid buffer, and Tris-HCl buffer, and the like, and suitable combinations of two or more hereof. Alternatively, the buffer is selected from the group consisting of sodium phosphate, sodium carbonate, sodium hydroxide, glutamate, glycine, Tris base buffers, and the like, and suitable combinations of two or more hereof.

In a further preferred embodiment of the method of preparing a formulation, the method also includes sterilizing the solution. Preferably, the sterilizing is done by filtration

Thus, a particular embodiment of the method of preparing a Formulation aspect of the invention features a method of preparing a formulation, the method comprising adding to a salt solution, formed in situ via addition of a molar equivalent of sodium hydroxide to 3-[2,4-dimethyl-5-(2-oxo-1,2-dihydro-indol-3-ylidenemethyl)-1H-pyrrol-3-yl]-propionic acid, polyethylene glycol 300 and a sodium phosphate buffer. In preferred embodiments of this method of preparing a formulation, the method also includes adding benzyl alcohol to the salt solution, and/or sterilizing the resulting formulation by filtration.

In an additional aspect, the invention features a method of preparing a formulation suitable for oral administration, the method comprising admixing an ionizable substituted indolinone, one or more pharmaceutically acceptable surfactants, and one or more pharmaceutically acceptable oils, wherein the ionizable substituted indolinone is 3-[2,4-dimethyl-5-(2-oxo-1,2-dihydro-indol-3-ylidenemethyl)-1H-pyrrol-3-yl]-propionic acid, and pharmaceutically active salts and derivatives thereof. In other preferred embodiments, each of the one or more pharmaceutically acceptable oils is a vegetable oil (e.g., sesame oil, and the like, as described above), and/or each of the one or more pharmaceutically acceptable surfactants is an ethylene oxide copolymer (e.g., PLURONIC™ F68, and the like, as described above).

Other components can also be added to the invention formulations to enhance their therapeutic effects. For example, the ionizable substituted indolinones may be further formulated in liposomes in addition to the above-mentioned components. Liposomes have been shown to enhance the delivery of compounds into cells. However, because the formulations have been shown to have a therapeutic effect with only the components described herein, formulations of the present invention may also "consist essentially of" or "consist of" these components.

In preferred embodiments of the invention, the formulations are effective in treating or preventing an abnormal condition in a patient in need of such treatment. The patient is preferably a mammal and more preferably a human. In a highly preferred embodiment, the formulations are parenteral. Parenteral administration includes intravenous, subcutaneous, intraperitoneal, intramuscular, intraosseous, and intramedullary injection, and the like, and suitable combinations of two or more hereof.

The term "preventing" as used herein refers to administering the formulation to a patient before the abnormal condition manifests itself in that patient.

The term "treating" as used herein refers to the use of the ionizable substituted indolinone having a therapeutic effect and at least partially alleviating or abrogating the abnormal condition in the organism (e.g., patient).

The term "therapeutic effect" as used herein refers to inhibition of the abnormal condition. The term "therapeutic effect" also refers to the inhibition of factors causing or contributing to the abnormal condition. A therapeutic effect relieves to some extent one or more of the symptoms of the abnormal condition.

The term "mammal" as used herein preferably refers to the organisms of the class known as "mammalia", such as mice, rats, rabbits, guinea pigs, goats, sheep, horses, cows, dogs, cats, monkeys, apes, humans, and the like; more preferably dogs, cats, monkeys, apes, humans, and the like; and most preferably humans.

The term "abnormal condition" refers to a function in the cells or tissues of a patient that deviates from normal functions in that patient. An abnormal condition can relate to cell proliferation (e.g., be a cell proliferative disorder) as described herein.

The term "cell proliferative disorder" as used herein refers to a disorder where an excess cell proliferation of one or more subset of cells in a multicellular organism occurs resulting in harm (e.g., discomfort or decreased life expectancy) to the multicellular organism. The excess cell proliferation can be determined by reference to the general population and/or by reference to a particular patient (e.g., at an earlier point in the patient's life). Hyper-proliferative cell disorders can occur in different types of animals and in humans, and produce different physical manifestations depending upon the affected cells. Hyper-proliferative cell disorders include without limitation cancers, blood vessel proliferative disorders, fibrotic disorders, autoimmune disorders, and the like. Cell proliferative disorders suitable for treatment in accordance with the present invention include without limitation cancers (e.g., erythroblastoma, glioblastoma, meningioma, astrocytoma, melanoma, myoblastoma, breast cancers, gastric cancers, ovarian cancers, renal cancers, hepatic cancers, pancreatic cancers, bladder cancers, thyroid cancers, prostate cancers, colorectal cancers, solid tumor cancers, colon cancer, brain cancer, blood cancers, bone cancers, liver cancer, kidney cancer, stomach cancer, lung cancer, Kaposi's sarcoma, non-small cell lung cancer, skin cancer, and the like, non-small cell lung cancers, and the like).

In reference to the treatment of abnormal conditions caused, in whole or in part, by a cell proliferative disorder, a therapeutic effect refers to one or more of the following: (a) reducing tumor size; (b) inhibiting (e.g, slowing or stopping) tumor metastasis; (c) inhibiting tumor growth; and (d) relieving to some extent one or more of the symptoms associated with the abnormal condition.

Thus, the present invention features a parenteral formulation comprising an ionizable substituted indolinone, one or more polyoxyhydrocarbyl compounds, and a buffer for preventing or treating an abnormal condition in a patient in need of treatment comprising: (a) diluting said parenteral formulation into a pharmaceutically acceptable solution; and (b) parenterally administering said diluted formulation to said patient, wherein the ionizable substituted indolinone is 3-[2,4-dimethyl-5-(2-oxo-1,2-dihydro-indol-3-ylidenemethyl)-1H-pyrrol-3-yl]-propionic acid, and pharmaceutically active salts and derivatives thereof.

In preferred embodiments thereof, the ionizable substituted indolinone is solubilized by combining with a molar equivalent of a base solution or an acid solution. Preferably, the base solution is selected from the group consisting of sodium hydroxide, ammonium hydroxide, triethylamine, ethylenediamine, N-methyl-D-glucamine, choline, triethanolamine, and the like, and suitable combinations of two or more hereof, and the acid solution is selected from the group consisting of hydrochloric acid, sulfuric acid, formic acid, lactic acid, malic acid, succinic acid, acetic acid, methane sulfonic acid, benzene sulfonic acid, phosphoric acid, and the like, and suitable combinations of two or more hereof.

In yet other preferred embodiments thereof, the one or more polyoxyhydrocarbyl compounds is selected from the group consisting of polyethylene glycol 300, polyethylene glycol 400, propyleneglycol, glycerin, and the like, and suitable combinations of two or more hereof. Preferably, the one or more polyoxyhydrocarbyl compounds is polyethylene glycol 300.

In other preferred embodiments thereof, the buffer pH is three pH units higher than the pka of said ionizable substituted indolinone, or three pH units lower than the pkb of said ionizable substituted indolinone. Preferably, the buffer has a molarity between 0.01 M and 0.1 M, and is selected from the group consisting of acetate, citrate, phosphoric acid buffer, ascorbate, hydrochloric acid buffer, Tris-HCl buffer, and the like, and suitable combinations of two or more hereof. Alternatively, the buffer is selected from the group consisting of sodium phosphate, sodium carbonate, sodium hydroxide, glutamate, glycine, Tris base buffers, and the like, and suitable combinations of two or more hereof. Preferably, the buffer is sodium phosphate buffer.

In other preferred embodiments thereof, the formulation also contains one or more pharmaceutically acceptable surfactants. Preferably, pharmaceutically acceptable surfactants include but are not limited to POLYSORBATE 80 and other polyoxyethylene sorbitan fatty acid esters, glyceryl monooleate, sorbitan monooleate, pluronic F68, lecithin, polyvinyl alcohol, ethylene oxide copolymers such as PLURONIC™ (a polyether) and TETRONIC™ (BASF), polyol moieties, sorbitan esters, and the like, and suitable combinations of two or more hereof. Preferably ethoxylated castor oils, such as CREMOPHOR EL^{®}, are used for the formulation of hydrophobic pharmaceutical agents. In other preferred embodiments thereof, the formulation also contains a pharmaceutically acceptable preservative. Preferably, the preservative is selected from the group consisting of benzyl alcohol, methyl paraben, ethyl paraben, phenol, and the like, and suitable combinations of two or more hereof. Most preferably, the preservative is benzyl alcohol.

In other preferred embodiments thereof, the formulation also contains an antioxidant. Preferably, the antioxidant is selected from the group consisting of sodium meta-bisulfite, EDTA, ascorbic acid, benzyl alcohol, and the like, and suitable combinations of two or more hereof, and preferably is benzyl alcohol.

In yet other preferred embodiments thereof, the patient is a mammal, preferably a human.

Further, the present invention features an oral formulation comprising an ionizable substituted indolinone, one or more pharmaceutically acceptable surfactants, and one or more pharmaceutically acceptable oils for preventing or treating an abnormal condition in a patient in need of treatment comprising orally administering to the patient the formulation wherein the ionizable substituted indolinone is 3-[2,4-dimethyl-5-(2-oxo-1,2-dihydro-indol-3-ylidenemethyl)-1H-pyrrol-3-yl]-propionic acid, and pharmaceutically active salts and derivatives thereof. In other preferred embodiments, each of the one or more pharmaceutically acceptable oils is a vegetable oil (e.g., sesame oil, and the like, as described above), and/or each of the one or more pharmaceutically acceptable surfactants is an ethylene oxide copolymer (e.g., PLURONIC™ F68, and the like, as described above).

The ionizable substituted indolinone is 3-[2,4-dimethyl-5-(2-oxo-1,2-dihydro-indol-3-ylidenemethyl)-1H-pyrrol-3-yl]-propionic acid (compound IV), and pharmaceutically active salts and derivatives thereof.

Other features and advantages of the invention will be apparent from the following description of the preferred embodiments and from the claims.

### DETAILED DESCRIPTION OF THE PREFERRED EMBODIMENTS

The instant invention features parenteral and oral formulations for indolinone compounds that are ionizable as free acids or free bases. In particular, the formulations aid the administration of indolinones that are ionizable as free acids or free bases to patients in need of treatment.

### 1. Properties of 3-[2,4-Dimethyl-5-(2-oxo-1,2-dihydro-indol-3-ylidenemethyl)-1H-pyrrol-3-yl]-propionic acid

3-[2,4-Dimethyl-5-(2-oxo-1,2-dihydro-indol-3-ylidenemethyl)-1H-pyrrol-3-yl]-propionic acid, is an indolinone compound that is a potent inhibitor of signal transduction via Flk-1/KDR, PDGF receptor, and FGF receptor. It is also efficacious in inhibiting SC growth of multiple tumor types and inhibited the growth of established tumors. This compound is a preferred compound for use in the formulations of the present invention. Properties of formulations comprising this compound are set forth in the experimental materials that follow.

### 2. PHARMACEUTICAL COMPOSITIONS AND USES

A compound or combination of the present invention or a physiologically acceptable salt of the compound can be administered as such to a human patient or it can be administered in pharmaceutical compositions or formulations in which the foregoing materials are mixed with suitable carriers or excipient(s). Techniques for formulation and administration of drugs may be found in "Remington's Pharmacological Sciences," Mack Publishing Co., Easton, PA, latest edition.

### A. Routes of Administration.

### General

Suitable routes of administration may include, without limitation, oral, rectal, transmucosal or intestinal administration or intramuscular, subcutaneous, intramedullary, intrathecal, direct intraventricular, intravenous, intraperitoneal, intranasal, or intraocular injections. The preferred routes of administration are oral and parenteral.

Alternatively, one may administer the compound in a local rather than systemic manner, for example, via injection of the compound directly into a solid tumor, often in a depot or sustained release formulation.

Furthermore, one may administer the drug in a targeted drug delivery system, for example, in a liposome coated with tumor-specific antibody. The Liposomes will be targeted to and taken up selectively by the tumor.

### B. Composition/Formulation

### General

Pharmaceutical compositions and formulations of the present invention may be manufactured by processes well know in the art; e.g., by means of conventional mixing, dissolving, granulating, dragee-making, levigating, emulsifying, encapsulating, entrapping or lyophilizing processes.

Pharmaceutical compositions and formulations for use in accordance with the present invention may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the compounds of the invention may be formulated in aqueous solutions, preferably in physiologically compatible buffers such as Hanks' solution, Ringers solution, or physiological saline bluffer. For transmucosal administration, penetrants appropriate to the barrier to be permeated are used in the formulation. Such penetrants are generally known in the art.

For oral administration, the compounds can be formulated by combining the active compounds with pharmaceutically acceptable carriers well known in the art. Such carriers enable the compounds of the invention to be formulated as tablets, pills, lozenges, dragees, capsules, liquids, gels, syrups, slurries, suspensions and the like, for oral ingestion by a patient. Pharmaceutical preparations and formulations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol; cellulose preparations such as, for example, maize starch, wheat starch, rice starch and potato starch and other materials such as gelatin, gum tragacanth, methyl cellulose, hydroxypropylmethylcellulose, sodium carboxymethylcellulose, and/or polyvinylpyrrolidone (PVP). If desired, disintegrating agents may be added, such as cross-linked polyvinylpyrrolidone, agar, or alginic acid. A salt such as sodium alginate may also be used.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions and formulations which can be used orally include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compounds may be dissolved or suspended in suitable liquids, such as fatty oils, liquid paraffin, or liquid polyethylene glycols. Stabilizers may be added in these formulations, also.

For buccal administration, the compositions and formulations may take the form of tablets or lozenges formulated in conventional manner.

For administration by inhalation, the compounds for use according to the present invention are conveniently delivered in the form of an aerosol spray using a pressurized pack or a nebulizer and a suitable propellant, e.g., without limitation, dichlorodifluoromethane, trichlorofluoromethane, dichlorotetra- fluoroethane or carbon dioxide. In the case of a pressurized aerosol, the dosage unit may be controlled by providing a valve to deliver a metered amount. Capsules and cartridges of, for example, gelatin for use in an inhaler or insufflator may be formulated containing a powder mix of the compound and a suitable powder base such as lactose or starch.

The compounds may also be formulated for parenteral administration, e.g., by bolus injection or continuous infusion. Formulations for injection may be presented in unit dosage form, e.g-, in ampoules or in multi-dose containers, with an added preservative. The compositions may take such forms as suspensions, solutions or emulsions in oily or aqueous vehicles, and may contain formulating materials such as suspending, stabilizing and/or dispersing agents.

Pharmaceutical compositions and formulations for parenteral administration include aqueous solutions of a water soluble form, such as, without limitation, a salt, of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances which increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions.

Alternatively, the active ingredient may be in powder form for constitution with a suitable vehicle, e.g., sterile, pyrogen-free water, before use.

The compounds may also be formulated in rectal compositions such as suppositories or retention enemas, using, e.g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

A non-limiting example of a pharmaceutical carrier for the hydrophobic compounds of the invention is a cosolvent system comprising benzyl alcohol, a nonpolar surfactant, a water-miscible organic polymer and an aqueous phase such as the VPD co-solvent system. VPD is a solution of 3% w/v benzyl alcohol, 8% w/v of the nonpolar surfactant Polysorbate 80™, and 65% w/v polyethylene glycol 300, made up to volume in absolute ethanol. The VPD co-solvent system (VPD:D5W) consists of VPD diluted 1:1 with a 5% dextrose in water solution. This co-solvent system dissolves hydrophobic compounds well, and itself produces low toxicity upon systemic administration, The proportions of such a co-solvent system may be varied considerably without destroying its solubility and toxicity characteristics. Furthermore, the identity of the co-solvent components may be varied: for example, other low-toxicity nonpolar surfactants may be used instead of Polysorbate 80™, the fraction size of polyethylene glycol may be varied; other biocompatible polymers may replace polyethylene glycol, e.g., polyvinyl pyrrolidone; and other sugars of polysaccharides may substitute for dextrose.

Alternatively, other delivery systems for hydrophobic pharmaceutical compounds may be employed. Liposomes and emulsions are well known examples of delivery vehicles or carriers for hydrophobic drugs. In addition, certain organic solvents such as dimethylsulfoxide also may be employed, although often at the cost of greater toxicity.

Additionally, the compounds and formulations may be delivered using a sustained-release system, such as semi-permeable matrices of solid hydrophobic polymers containing the therapeutic agent. Various sustained-release materials have been established and are well known by those skilled in the art.

The pharmaceutical compositions and formulation herein also may comprise suitable solid or gel phase carriers or excipients. Examples of such carriers or excipients include, but are not limited to, calcium carbonate, calcium phosphate, various sugars, starches, cellulose derivatives, gelatin, and polymers such as polyethylene glycols.

The compound of the invention may be provided as a salt with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents than are the corresponding free base forms.

### 3-[2,4-Dimethyl,-5-(2-oxo-1,2-dihydro-indol-3-ylidenemethyl)-1H pyrrol-3-yl]-propionic acid composition

This compound may be formulate as any of the compositions and formulations described above. Presently preferred formulations, however, comprise 3-[2,4-Dimethyl-5-(2-oxo-1,2-dihydro-indol-3-ylidenemethyl)-1H-pyrro1-3-yl]-propionic acid composition in sufficient sterile parenteral solution to afford a final concentration of about 10 mg/ml.

### 3. DOSAGE

### A. General

Compounds, combinations, and pharmaceutical compositions and formulations suitable for use in the present invention include compositions wherein the active ingredients are contained in an amount sufficient to achieve the intended purpose; i.e., the modulation of protein kinases (PK) activity or the treatment or prevention of a PK-related disorder.

The above referenced protein kinase related disorder is selected from the group consisting of an EGFR related disorder, a PDGFR related disorder, an IGFR related disorder and a flk related disorder.

The above referenced protein kinase related disorder is a cancer selected from the group consisting of squamous cell carcinoma, sarcomas such as Kaposi's sarcoma, astrocytoma, glioblastoma, lung cancer, bladder cancer, colorectal cancer, gastrointestinal cancer, head and neck cancer, melanoma, ovarian cancer, prostate cancer, breast cancer, small-cell lung cancer and glioma in a further aspect of this invention.

The above referenced protein kinase related disorder is selected from the group consisting of diabetes, a hyper- proliferation disorder, von Hippel-Lindau disease, restenosis, fibrosis, psoriasis, osteoarthritis, rheumatoid arthritis, an inflammatory disorder and angiogenesis in yet another aspect of this invention.

Additional disorders which may be treated or prevented using the compounds of this invention are immunological disorders such as autoimmune disease (AIDS) and cardiovasular disorders such as atherosclerosis.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any compound used according to the invention, the therapeutically effective amount or dose can be estimated initially from cell culture assays. Then, the dosage can be formulated for use in animal models so as to achieve a circulating concentration range that includes the IC₅₀ as determined in cell culture (i,e., the concentration of the test compound which achieves a half-maximal inhibition of the PK activity). Such information can then be used to more accurately determine useful doses in humans.

Toxicity and therapeutic efficacy of the compounds described herein can be determined by standard pharmaceutical procedures in cell cultures or experimental animals, e.g., by determining the IC₅₀ and the LD₅₀ (both of which are discussed elsewhere herein) for a subject compound. The data obtained from these cell culture assays and animal studies can be used in formulating a range of dosage for use in humans. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1.

Therapeutic compounds should be more potent in inhibiting preceptor tyrosine kinase activity than in exerting a cytotoxic effect A measure of the effectiveness and cell toxicity of a compound can be obtained by determining the therapeutic index; i.e., IC₅₀/LD_{50.} IC₅₀, the dose required to achieve 50% inhibition, can be measured using standard techniques such as those described herein. LD₅₀, the dosage which results in 50% toxicity, can also be measured by standard techniques as well(Mossman, 1983, J. Immunol. Methods. 65-55-63), by measuring the amount of LDH released (Korzeniewski and Callewaert, 1983, J. Immunol. Methods, 64:313; Decker and Lohmann-Matthes, 1988, J. Immunol. Methods, 115:61), or by measuring the lethal dose in animal models. Compounds with a large therapeutic index are preferred. Thus, in one aspect of the invention, a preferred dosage of the compounds, agents, combinations, and pharmaceutical compositions contemplated for use in the invention requires the therapeutic index of each active component to be greater than 2, preferably, at least 10, more preferably, at least 50.

Dosage amount and interval may be adjusted individually to provide plasma levels of the active species, which are sufficient to maintain the kinase modulating effects. These plasma levels are referred to as minimal effective concentrations (MBCs). The MEC will vary for each compound but can be estimated from in vitro data; e.g., the concentration necessary to achieve 30-90% inhibition of a kinase may be ascertained using the assays described herein. Dosages necessary to achieve the MEC will depend on individual characteristics and route of administration. HPLC assays or bioassays can be used to determine plasma concentrations.

Dosage-intervals can also be determined using MEC value. Compounds should be administered using a regimen that maintains plasma levels above the MEC for 10-90% of the time, preferably between 30-90% and most preferably between 50-90%.

In cases of local administration or selective uptake, the effective local concentration of the drug may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

### B- 3-[2,4-Dimethyl-5-(2-oxo-1,2-dihydro-indol-3-ylidenemethyl)-1H-pyrrol-3-yl]-propionic Acid.

### Therapeutically Effective Amounts

In general, a "therapeutically effective amounts" refers to that amount of an agent or its metabolite which is effective to prevent, alleviate, reduce or ameliorate symptoms of disease and/or the undesired side effects attributable to treatment of disease with another agent or its metabolite, or to prolong the survival of the patient being treated. More particularly, in reference to the treatment of cancer, a therapeutically effective amount refers to that amount which has the effect of (1) reducing the size of (or preferably eliminating) the tumor; (2) inhibiting (that is, slowing to some extent, preferably stopping) tumor metastasis; (3) inhibiting to some extent (that is slowing to some extent, preferably stopping) tumor growth; and/or, (4) relieving to some extent (or preferably eliminating) one or more symptoms associated with the cancer and/or one or more undesired side effects attributable to treatment of the cancer with another agent or its metabolite. Non-limiting examples of therapeutically effective amounts of particular agents and compounds contemplated for use in the present invention are further described below,

In addition to the above general definition, by a "therapeutically effective amount" of an agent is meant any amount administered in any manner and in any treatment regime as may be currently recognized in the medical arts or as may come about as the result of future developments regarding the use of these agents.

A "treatment regime" refers to specific quantities of the ionizable substituted indolinone contemplated for use in this invention) administered at set times in a set manner over an established time period.

When referring to "set times" of administration within a treatment regime, "consecutive days" means consecutive calendar days; i.e., Monday, Tuesday, Wednesday, etc. "Staggered" days means calendar days with other calendar days between them, e.g., without limitation, Monday, Wednesday, Saturday, etc.

Furthermore, with regard to a "therapeutically elective amount" of an ionizable substituted indolinone, the phrase refers to an amount of the compound sufficient to inhibit the growth, size and vascularization; i.e., angiogenesis and/or vasculogenesis, of tumors during the "recovery" period, i.e., the periods in a treatment regime when no other chemotherapeutic agent is being administered to a patient.

### Specific Amounts

Based on the pharmacological data obtained regarding 3-[2,4-Dimethyl-5-(2-oxo-1,2-dihydro-indol-3-ylidonemethyl)-1H-pyrrol-3-yl]-propionic acid composition (see above), the compound may be administered in doses ranging from about 1 mg/m² to about 3000 mg/m². In a presently preferred embodiment, the dosage is between about 50 mg/m² and about 2400 mg/m². In another preferred embodiment, therapeutically effective amounts of 3-[2,4-Dimethyl-5-(2-oxo-1,2-dihydm-indol-3-ylidenemethyl)-1H-pyrrol-3-yl]-propionic acid composition comprise from about 50 to about 800 mg/m². Of course, the dose would depend on a number os factors, including patient specific factor, e.g., weight, dosing regimen (e.g., frequency, route of administration, effect of food) etc.

The formulation(s) described in the above composition section may be administered to a patient at a rate of from about 0.1 to about 200 cc/hour. The rate of administration for a particular patient is dependent on achieving therapeutically relevant plasma levels for the particular indication. The prescribing physician is skilled in making such as determination.

By "about," wherever the term appears herein, is meant ±10%; i.e., about 175 cc/hour means from 157.5 cc/hour to 192.5 cc/hour, etc.

In a presently preferred embodiment, the 3-[2,4-Dimethyl-5-(2-oxo-1,2-dihydra-indol-3-ylidenemethyl)-1H-pyrrol-3-yl]-propionic acid composition dose is administered during rest periods when no other agent is being administered to a patient.

### B. Pharmaceutically Acceptable Carriers.

The following tables set forth the ranges of components useful in the formulation of the compounds disclosed herein.

### All formulation components:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10% | 0.01-7.5% | 0.01-5.0% |
| Polyoxyhydrocarbyl Compounds | 5.0-70% | 5.0-45% | 15-45% |
| Buffer | 0-3% | 0-1.0% | 0-0.5% |
| Surfactant | 0-50.0% | 0-50.0% | 0-31.5% |
| preservative | 0-3.0% | 0-2.0% | 0.5-2.0% |
| Antioxidant | 0-3.0% | 0-2.0% | 0.1-1.0% |
| Alcohol | 0-40.0% | 0-30.0% | 0-25.0% |
| Oil | 5-75% | 10-25% | 15-20% |
| Granulating Agent | 10-95% | 20-60% | 30-50% |
| Diluent | 5-95% | 10-80% | 20-60% |
| Binder | 0-15% | 0-8% | 0-5% |
| Disintegrant | 1-20% | 4-15% | 4-10% |
| Lubricant | 0.3-2.0% | 0.5-1,5% | 1.0-1.5% |
| Flow Enhancer | 0-1.0% | 0.3-1.0% | 0.3.0.8% |
| Suspending Agent | 0-2.0% | 0-1.0% | 0-0.5% |

### Indolinone + Polyoxyhydrocarbyl Compounds:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10.0% | 0.01-7.5% | 0.01-5.0% |
| Polyoxyhydrocarbyl Compounds | 1.0-70.0% | 5.0.45% | 15.0-45.0% |

### lndolinone + Buffer:

| Component | Broadest Range of Component Concentration in Formulation | preferred Range of Component Concentration in Formulation | Most Preferred Range of Component Concentration in Formulation |
|---|---|---|---|
| Ionizable Substituted Indolinone | 0.01-10.0% | 0.01-7.5% | 0.01-5-0% |
| Buffer | 0,01M-1M | 0.05-0.5M | 0.2-0.5M |

### Indolinone + Preservative:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10.0% | 0.01-7.5% | 0.01-5.0% |
| Preservative | 0-3.0% | 0-2.0% | 0.5-2.0% |

### Indolinone + Antioxidant:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.41-10.0% | 0.0-7.5% | 0.01-3.0% |
| Antioxidant | 0-3.0% | 0-2.0% | 0.1-1,0% |

### Indolinone + Alcohol:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10% | 0.01-7.5% | 0.01-5.0% |
| Alcohol | 0-40.0% | 0-30.0% | 0-25.0% |

### Indolinone + Oil:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 5-75% | 10-25% | 15% |
| Oil | 20-90% | 70-85% | 80% |

### Indolinone + Surfactant:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10.0% | 0.01-7.5% | 0.01-5.0% |
| Surfactant | 0-50.0% | 0-40.0% | 0-31.5% |

### Indolinone + Granulating Agent:

| Component | Broadest Range of Component Concentration in Formulation | Preferred Range of Component Concentration in Formulation | Most Preferred Range or Component Concentration in Formulations |
|---|---|---|---|
| Ionizable Substituted Indolinone | 5-90% | 40.80% | 50-70% |
| Granulating Agent | 10-95% | 20-60% | 30-50% |

### Indolinone + Polyoxyhydrocarbyl Compounds + Surfactant + Polyglycolized Lipids:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10.0% | 0.01-7.5% | 0.01-5.0% |
| Polyoxyhydrocarbyl Compounds | 5.0-70% | 5-45% | 1 5.0-45.0% |
| Surfactant | 0-50% | 0-40% | 0-31.5 |

### Indolinone + Surfactant + Diluent + Binder + Disintegrant + Lubricant + Flow Enhancer:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 5-90% | 10-80% | 15-75% |
| Surfactant | 0-10% | 0.1-8.0% | 1-5% |
| Blinder | 5-95% | 15-85% | 20-75% |
| Disintegrant | 1-20% | 4-15% | 4-10% |
| Lubricant | 0.3-2.0% | 0.5.1.5% | 1.0-1.5% |
| Flow Enhancer | 0-1.0% | 0.3-1.0% | 0.3-0.8% |

### Indolinone + Polyoxyhydrocarbyl Compounds + Buffer + Surfactants:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01.10.0% | 0.01-7.5% | 0.01-5.0% |
| Polyoxyhydrocarbyl Compounds | 5-70% | 5-45% | 15-45% |
| Buffer | 0-3% | 0-1% | 0-0.5% |
| Surfactant | 0-50% | 0-40% | 0-31.5% |

### Indolinone + Surfactant + Salt Solution + Suspending Agent:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 0.01-10% | 0.01-7.5% | 0.01-5.0% |
| Surfactant | 0-50% | 0.1-1.0% | 0.1-0.5% |
| Salt Solution | 0.5-1% | 0.5-1% | 0.5-1% |
| Suspending Agent | 0-2% | 0-1% | 0-0.5% |

### Indolinone + Surfactant + Oil:

| Component | Broadest Range of Component Concentration in Formulation | Preferred Range of Component Concentration in Formulation | Most Preferred Range of Component Concentration in Formulation |
|---|---|---|---|
| Ionizable Substituted Indolinone | 10-30% | 10-25% | 10-20% |
| Surfactants | 0.10% | 3-8% | 5% |
| Oil | 50-80% | 60-80% | 70-80% |

### Method of Making Formulation comprising Indolinone + Polyoxyhydrocarbyl Compounds + Buffer:

| Component | Broadest Range of Component Concentration in Formulation Made By Method | Preferred Range of Component Concentration in Formulation Made By Method | Most Preferred Range of Component Concentration in Formulation Made By Method |
|---|---|---|---|
| lonizable Substituted Indolinone | 0.01-10% | 0.01-7.5% | 0.01-5% |
| Polyoxyhydrocarbyl Compounds | 5-70% | 5-50% | 5-45% |
| Buffer | 0-3% | 0-1% | 0-0.05% |

### Method of Making Formulation comprising Indolinone + Surfactant + Oil:

| Component | Broadest Range of Component Concentration in Formulation Made By Method | Preferred Range of Component Concentration in Formulation Made By Method | Most Preferred Range of Component Concentration in Formulation Made By Method |
|---|---|---|---|
| Ionizable Substituted | 1-1000 mg | 10-900 mg | 50-750 mg |
| Indolinone | | | |
| Surfactant | 1-100 mg | 10-90 mg | 45-55 mg |
| Oil | 10-1000 mg | 10-900 mg | 200-900 mg |

### 4. PACKAGING

The compositions may, if desired, be presented in a pack or dispenser device, such as an FDA approved kit, which may contain one or more unit dosage forms containing the active ingredient. The pack may for example comprise metal or plastic foil, such as a blister pack. The pack or dispenser device may be accompanied by instructions for administration. The pack or dispenser may also be accompanied by a notice associated with the container in a form prescribed by a governmental agency regulating the manufactured, use or sale of pharmaceuticals, which notice is reflective of approval by the agency of the form of the compositions or of human or veterinary administration. Such notice, for example, may be of the labeling approved by the U.S. Food and Drug Administration for prescription drugs or of an approved product insert. Compositions comprising a compound of the invention formulated in a compatible pharmaceutical carrier may also be prepared, placed in an appropriate container, and labeled fur treatment of an indicated condition. Suitable conditions indicated on the label may include treatment of a tumor, inhibition of angiogenesis, treatment of fibrosis, diabetes, and the like.

Additional methods of preparing pharmaceutical formulations of the compounds, methods of determining the amounts of compounds to be administered to a patient, and modes of administering compounds to an organism are disclosed in U.S. Application Serial No. 08/702,232 by Tang, et al., and entitled "Indolinone Combinatorial Libraries and Related Products and Methods for the Treatment of Disease," filed August 23, 1996, and International patent publication number WO 96/22976, by Buzzetti, et al., and entitled "Hydrosoluble 3-Arylidene-2-Oxindole Derivatives as Tyrosine kinase Inhibitors," published August 1, 1996, both of which are incorporated herein by reference in their Entirety, including any drawings. Those skilled in the art will appreciate that such descriptions are applicable to the present invention and can be easily adapted to it.

### SYNTHESIS EXAMPLES

The compounds of this invention, as well as the precursor 2-oxindoles and aldehydes, may be readily synthesized using techniques well known in the chemical arts. It will be appreciated by those skilled in the art that other synthetic pathways for forming the compounds of the invention are available and that the following is offered by way of example and not limitation.

### A. General synthetic procedure.

The following general methodology may be employed to prepare the compounds of this invention:

The appropriately substituted 2-oxindole (1 equiv.), the appropriately substituted aldehyde (1.2 equiv.) and a base (0.1 equiv.) are mixed in a solvent (1-2 ml/mmol 2-oxindole) and the mixtures is then heated for from about 2 to about 12 hours. After cooling, the precipitate that forms is filtered, washed with cold ethanol or ether and vacuum dried to give the solid product. If no precipitate forms, the reaction mixture is concentrated and the residue is triturated with dichloromethane/ether, the resulting solid is collected by filtration and then dried. The product may optionally be further purified by chromatography.

The base may be an organic or an inorganic base. If an organic base is used, preferably it is a nitrogen base. Examples of organic nitrogen bases include, but are not limited to, diisopropylamine, trimethylamine, triethylamine, aniline, pyridine, 1,8.diazabicyclo[5.4.1]undec-7-ene, pyrrolidine and piperidine.

Examples of inorganic bases are, without limitation, ammonia, alkali metal or alkaline earth hydroxides, phosphates, carbonates, bicarbonates, bisulfates and amides. The alkali metals include, lithium, sodium and potassium while the alkaline earths include calcium, magnesium and barium.

In a presently preferred embodiment of this invention, when the solvent is a protic solvent, such as water or alcohol, the base is an alkali metal or an alkaline earth inorganic base, preferably, a alkali metal or an alkaline earth hydroxide.

It will be clear to those spilled in the art, based both on know general principles of organic synthesis and on the disclosures herein which base would be most appropriate for the reaction contemplated.

The solvent in which the reaction is carried out may be a protic or an aprotic solvent, preferably it is a protic solvent. A "aprotic solvent" is a solvent which has hydrogen atom(s) covalently bonded to oxygen or nitrogen atoms which renders the hydrogen atoms appreciably acidic and thus capable of being "shared" with a solute through hydrogen bonding. Examples of protic solvents include, without limitation, water and alcohols.

An "aprotic solvent" may be polar or non-polar but, in either case, does not contain acidic hydrogens and therefore is not capable of hydrogen bonding with solutes. Examples, without limitation, of non-polar aprotic solvents, are pentane, hexane, benzene, toluene, methylene chloride and carbon tetrachloride. Examples of polar aprotic solvents are chloroform, tetrahydro- furan, dimethylsulfoxide and dimethylformamide.

In a presently preferred embodiment of this invention, the solvent is a protic solvent, preferably water or an alcohol such as ethanol.

The reaction is carried out at temperatures greater than room temperature. The temperature is generally from about 30° C to about 150° C, preferably about 80°C to about 100° C, most preferable about 75° C to about 85° C, which is about the boiling point of ethanol. By "about" is meant that the temperature range is preferably within 10 degrees Celcius of the indicated temperature, more preferably within 5 degrees Celcius of the indicated temperature and, most preferably, within 2 degrees Celcius of the indicated temperature. Thus, for example, by "about 75° C" is meant 75° C± 10° C, preferably 75° C± 5° C and most preferably, 75° C±2° C.

### B. Synthetic methods used in the examples which follow:

### Method A: Formylatlon of pyrroles

POCl₃ (1.1 equiv.) is added dropwise to dimethylformamide (3 equiv.)at-10° C followed by addition of the appropriate pyrrole dissolved in dimethylformamide. After stirring for two hours, the reaction mixture is diluted with H₂O and basified to pH 11 with 10 N KOH. The precipitate which forms is collected by filtration, washed with H₂O and dried in a vacuum oven to give the desired aldehyde.

### Method B: Saponification of pyrrolecarboxylic acid esters

A mixture of a pyrrolecarboxylic acid ester and koh (2-4 equiv.) In etoh is refluxed until reaction completion is indicated by thin layer chromatography (tlc). The cooled reaction mixture is acidified to ph 3 with 1 n hcl. The precipitate which formes is collected by filtration, washed with h₂O and dried in a vacuum oven to give the desired pyrrolecarboxylic acid.

### Method C: Amidation

To a stirred solution of a pyrrolecarboxylic acid dissolved in dimethylformamide(0.3M) is added 1-ethyl-3-(3-dimethylaminopropyl)carbodiimide (1.2 equiv.), 1-hydroxybenzotriazole (1.2 equiv.), and triethylamine (2 equiv.). The appropriate amine is added (1 equiv.) and the reaction stirred until completion is indicated by TLC. Ethyl acetate is then added to the reaction mixture and the solution washed with saturated NaHCO₃ and brine (with extra salt), dried over anhydrous MgSO₄ and concentrated to afford the desired amide.

### Method D: Condensation of aldehydes and oxindoles containing carboxylic acid substituents

A mixture of the oxindole (1 equivalent), 1 equivalent of the aldehyde and 1 - 3 equivalents of piperidine (or pyrrolidine) in ethanol (0.4 M) is stirred at 90-100° C until reaction completion is indicated by TLC. The mixture is then concentrated and the residue acidified with 2N HCl. The precipitate that forms is washed with H₂O and EtOH and then dried in a vacuum oven to give the product.

### C. Synthesis of pyrrole substituted 2-indolinones

### Example 1 (from Table 1)

### 3-[2,4-Dimethyl-5-(2-oxo-1,2-dihydroindol-3-ylidenemethyl)-1H-pyrrolo-3-yl]-propionic acid

Sodium metal (1.5 g) was placed in a 3 L 3-neck round bottom flask equipped with a thermometer, reflux condenser and mechanical stirring and placed in an oil bath. Absolute ethanol (1 L) was added with stirring. When the sodium had dissolved, 350 g of pentan-2,4-dione was added all at once and then 310 g of ethyl acrylate added over 30 minutes. The mixture was refluxed for 2.5 hours and then allowed to cool to room temperature overnight. Glacial acetic acid (3 mL) was added and the solvent removed by rotary evaporation. The residue was filtered through a pad of diatomaceous earth and distilled in a wiped film still at 0-1 mm. The distillate was redistilled using a 10 inch vacuum jacketed Vigreux column to give 518 g of ethyl 5-acetyl-4-oxohexanoate, BP 84 - 92 °C at 0.2 - 0.7 mm.

To a 5 L three-neck flask equipped with a thermometer and a mechanical stirrer and heated on a steam bath was added 350 g ethyl 5-acetyl-4-oxohexanoate, 329 g ethyl aminomalonate hydrochloride, 133 g sodium acetate and 1.2 L acetic acid. The mixture was heated to 99 °C over 37 minutes. By 62°C, carbon dioxide evolution was already rapid. After a total of 35 minutes at 99 °C gas, CO₂ evolution had greatly slowed. After another hour, the mixture was cooled, sodium chloride removed by vacuum filtration, and the solvent evaporated. The residue was mixed with 1 L of cold water. The precipitate was collected by vacuum filtration, washed with 400 mL water, and dissolved in 1 L of hot 95 % ethanol. The solution was treated with 20 g of Darco G-60, hot-filtered, and cooled to room temperature. The crystalline solid was collected by vacuum filtration, washed twice on the filter with 200 mL of 50 % ethanol and dried under vacuum at 70°C to give 285 g (64 % yield) of 2-ethoxycarbonyl-4-(2-ethoxycarbonylethyl)-3,5-dimethylpyrrole. The filtrate was refrigerated overnight to give another 53.1 g (11.9 % yield) of product for a total yield of 75.9%).

2,4-Dimethyl-5-ethoxycarbonyl-3-(2-ethoxycarbonylethyl)-pyrrole (1.07 kg) and 3.2 L 5 N sodium hydroxide were mechanically stirred in a 12 L three-neck round bottom flask equipped with a reflux condenser and an addition funnel and heated in an oil bath. The mixture was refluxed for 3 hours after which time the internal temperature was 96°C, all solids were dissolved and thin layer chromatography showed hydrolysis to be complete. The heating bath was removed and the mixture cooled to 50°C in a water bath 12N Hydrochloric acid (~1.3 L) was slowly added. After about 50% of the acid was added gas evolution began and the temperature reached 60 °C. As more acid was added, gas evolution increased and a yellow precipitate formed. The final pH was adjusted to 3.5 with hydrochloric acid. The mixture was cooled in an ice bath to 8 °C. The solids were collected by vacuum filtration, washed twice with 0.5 L of distilled water and dried for 48 hours in a vacuum oven at 55-60 °C to give 677 g (101% yield) of 3-(2-carboxyethyl)-2,4-dimethylpyrrole.
¹HNMR (d₆-DMSO) δ 11.9(s,1H, COOH), 9.9(s, 1H, NH), 6.2(s, 1H, aromatic), 2.5 (t, 2H, CH₂), 2.2 (t, 2H, CH₂), 2.0(s, 3H, CH₃), 1.9(s, 3H, CH₃); MP 134-136°C.

Dimethylformamide (28.5 g) in 250 mL of dichloromethane in a 1 L three neck round bottom flask equipped with magnetic stirring, a thermometer and a dropping funnel was cooled in an ice-salt bath to -1 °C. Phosphorus oxychloride (59-3 g) was placed in the dropping funnel and slowly added to the reaction mixture. The funnel was flushed with 25 mL of dichloromethane to be sure all the phosphorus oxychloride. The maximum temperature reached by the mixture was 5 °C. The mixture was stirred for 1 minutes at which time the temperature was -3 °C. Solid 3-(2-Carboxyethyl)-2,4-dimethylpyrrole (32.6 g) was added in portions over 15 minutes. The maximum temperature reached by the mixture was 7°C. The reddish-black mixture was stirred for 30 minutes more and then heated to reflux for 1 hour. The mixture was cooled to 15 °C and 300 mL of water was added leading to a vigorous reaction during which the temperature increased. The mixture was stirred and cooled to 22°C and the layers separated and saved. The organic layer was extracted with 100 mL of water and the aqueous layers combined and washed with 50 ml of dichloromethane. The organic layers were discarded. The aqueous layer was adjusted to pH 11 with ~180 mL of 10 N sodium hydroxide. The temperature increased to 40 °C. The mixture was stirred for 30 minutes at which time the temperature was 27 °C. The mixture was acidified to pH 2 with ~120 mL) of 10 N hydrochloric acid which increased the temperature to 30 °C. Ethyl acetate (150 mL) was added and the mixture was stirred to extract the product. During stirring a considerable amount of black solid appeared on top of the water layer. The ethyl acetate layer was separated and the aqueous layer and solid was extracted twice with 100 mL of ethyl acetate. The solid still present was collected by vacuum filtration, washed thoroughly with water and dried under vacuum at 40 °C to give 12 g (31 % yield) of 3-(2-carboxyethyl)-2,4-dimethyl-5-formylpyrrole as a brownish-black solid. Thin layer chromatography (dichloro-methane:acetic acid, 95:5, silica gel) showed a spot at Rf 0.7 and a colored spot at the origin. The ethyl acetate layers were combined, dried over anhydrous sodium sulfate, and evaporated to a brownish-black solid which was dried under vacuum at 40 °C to give 21 g (55 % yield, total yield 86 %) of 3-(2-carboxyethyl)-2,4-dimethyl-5-formylpyrrole, identical in appearance to the previous solid by thin layer chromatography.

Alternatively, dimethylformamide (124 mL) in 750 mL of dichloromethane in a 5 L three-neck round bottom flask equipped with mechanical stirring, a thermometer and a dropping funnel was cooled in an ice-salt bath to -9 °C. Phosphorus oxychloride (114 mL) was added rapidly via the dropping funnel which was flushed into the reaction mixture with 50 mL of dichloromethane. The maximum temperature reached by the mixture was -4 °C. Solid 3-(2-carboxyethyl)-2,4-dimethylpyrrole (133.6 g) was added in portions over 20 minutes. The maximum temperature reached by the mixture was 3°C. The dark reddish mixture was heated to reflux for 1 minute and then cooled to -1°C. The mixture was cooled to 1 °C and 800 mL of ice water was rapidly added. The maximum temperature reached was 15 °C. The organic layer was separated and discarded. The aqueous layer was slowly adjusted to pH 12 - 13 with -800 mL of 10 N potassium hydroxide, adding ice to control the temperature. The temperature increased to 37 °C. The mixture was stirred for 90 minutes at ambient temperature at which time thin layer chromatography showed only a trace of light-colored material at the origin with the product at Rf 0.3. The mixture was cooled to 0 °C. The mixture was acidified to pH 3 with ~600 mL of 10 N hydrochloric acid ice being added to control the temperature. The maximum temperature reached was 10 °C. The mixture was stirred for 1 hour in the cold. The solid was collected by vacuum filtration, washed 4 times with 100 mL of water and dried under vacuum at 50 - 60 °C to give 140.6 g (90 % yield) of 3-(2-carboxyethyl-2,4-dimethyl-5-formylpyrrole as a brown solid.
¹HNMR (d₆-DMSO): δ 12.0(s, 1H, COOH), 11.3(s, 1H, NH), 9.4(s, 1H, CHO), 2.6 (t, 2H, CH₂), 2.3 (t,2H, CH₂), 2-2(s, 3H, CH₃), 2.1(s, 3H, CH₃). MP 145-147 °C.

3-(2-Carboxyethyl)-2,4-dimethyl-5-formylpyrrole (18.2 g) and 11-7 g 2-oxindole were dissolved in 100 mL of ethanol by heating in a 250 mL round bottom flask equipped with a magnetic stirrer and a reflux condenser in an oil bath. Pyrrolidine (7.0 g) was added and the reaction mixture refluxed for 2 hours at which time a large quantity of brown-black solid was present. Thin layer chromatography (ethyl acetate:ethanol:acetic acid 96:2:2, silica gel) showed the absence of oxindole starting material. Eight mL of acetic acid was added and the mixture refluxed for 15 minutes. The thick mixture was diluted with 50 mL of ethanol and cooled to 10 °C. The solid was collected by vacuum filtration and washed with 50 mL of ethanol. The solid was stirred in 125 ml of ethanol at reflux for 10 minutes, cooled to 10 °C, collected by vacuum filtration and washed with 50 mL of ethanol. The product was dried overnight at 45 °C under vacuum to give 25.5 g (88 % yield) of 3-(2,4-dimethyl-3-(2-carboxyethyl) pyrrol-5-methylidenyl]-2-indolinone as an orange solid.

Alternatively, a mixture of 3-(5-formyl-2,4-dimethyl-1H-pyrrol-3-yl)-propionic acid (10 g, 51 mmol), 2-oxindole (6.5 g, 49 mmol) and sodium hydroxide (40 g, 58 mmol) dissolved in 50 ml of water was stirred at 50 °C for 4 hours. The reaction mixture was cooled to room temperature, filtered and the filtrate acidified with to pH 3 with 12 N hydrochloric acid. The solid which precipitated was collected by vacuum filtration, washed with 10 ml of water and dried under vacuum overnight. The crude solid slurry washed with hot ethanol twice. The solid was then collected by vacuum filtration, washed with 10 ml of ethanol and dried under vacuum to give 13.8 g (91 %) of 3-[2,4-4imethyl-3-(2-oxo-1-dihydro-indol-3-ylidenemethyl)-1H-pyrrol-3-yl]-propionic acid.
¹HNMR (360 MHz, DMSO-d6): δ 13.38 (s, br, 1H, NH-1'), 12.05 (s, br,1H, COOH), 10.70 (s, br, 1H, NH-1), 7.69 (d, *J* = 7.39Hz, 1H, H-4), 7.53 (s, 1H, H-vinyl), 7.06 (t, *J*=7.39 Hz, 1H, H-6), 6.95 (t, *J*= 7.39 Hz, 1H, H-5), 6.85 (d. *J*= 7.39 Hz, 1H, H-7), 2.63 (t, *J*= 7.45 Hz, 2H, CH₂CH₂COOH), 2.34 (t, *J*= 7.45 Hz, 2H, CH₂CH₂COOH), 2.28 (s, 3H, CH₃), 2.24 (s, 3H, CH₃); MS m/z (relative intensity, %) 311 ([M+1]⁺, 100).

### Example 2 (from Table 1)

### 3-[2,4-Dimethyl-5-(2-oxo-1,2-dihydroindol-3-ylidenemethyl)-1H-pyrrol-3-yl]-propionic acid, sodium salt

A suspension of 8 g of 3-[2,4-dimethyl-5-(2-oxo-1,2-dihydroindol-3-ylidenemethyl)-1*H*-pyrrol-3-yl]-propionic acid in 60 mL of water was added to 0.98 g of sodium hydroxide in 10 ml of water. The mixture was stirred at RT for 30 minutes and filtered. The filtrate was frozen and lyophilized to give 8 g of the title compound.

Alternatively, a suspension of 117g of 318-005 in 470 mL of water was added to 16.47 g of sodium hydroxide in 74 mL water. The mixture was stirred at room temperature for 15 minutes and filtered. The filtrate was added to 210 mL of ethanol and the resulting precipitate which formed was collected by suction filtration. After drying, a total of 106 g of the title compound was obtained.
¹HNMR (360 MHz, DMSO-d6): δ 13.34 (s, br, 1H, NH-1'), 10.82 (s, br, 1H, NH-1), 7.65 (d, *J*=7-52Hz, 1H, H-4), 7.5 (s,1H, H-vinyl), 7.04 (t, *J*=7.52 Hz,1H, H₋6), 6.93 (t, *J*= 7.52 Hz, 1H, H-5), 6.85 (d, *J*= 7.52 Hz, 1H, H-7), 2.55 (t, *J*=6.95 Hz, 2H, CH₂CH₂COOH), 2.28 (s, 3H, CH₃), 2.24 (s, 3H, CH₃), and 1.99 (t, *J*= 6.95 Hz, 2H, CH₂CH₂COOH).

### BIOLOGICAL EVALUATION EXAMPLES

The examples given below are not limiting and are merely representative of various aspects and features of the present invention. The examples demonstrate parenteral and oral formulations, methods of making such formulations and uses of the formulations. The ionizable free acid, 3-[2,4-Dimethyl-5-(2-oxo-1,2-dihydro-indol-3-ylidenemethyl)-1H-pyrrol-3-yl]-propionic acid, is used as a representative example for the genus of ionizable free acids and free bases.

### EXAMPLE 1: In vivo Efficacy of 3-[2,4-Dimethyl-5-(2-oxo-1,2-dihydro-indol-3-ylidenemethyl)-1H-pyrrol-3-yl]-propionic acid

The ability of 3-(2,4-Dimethyl-5-(2-oxo-1,2-dihydro-indol-3-ylidenemethyl)-1H-pymol-3-yl]-propionic acid to inhibit subcutaneous (SC) growth of multiple tumor types in athymic mice was evaluated.

### Experimental Procedures

### Tumor Cell Lines

C6, SKOV3 and A431 cell lines were purchased from the American Type Culture Collection (Rockville, MD). SF763 and SF767 cells were obtained from Dr. Michael Berens (Barrow Neurological Institute). SF763T, SF767T, and SKOV3TP5 are tumor-derived sublines of SF763, SF767, and SKOV3 cells, respectively.

SF763T, SF767T, and SKOV3TP5 were derived by implanting the parental cells SC into BALB/c nu/nu mice. Tumors that displayed desirable growth characteristics were resected and finely minced in a sterile petri dish.

For the SF763T and SF767T cell lines, two to five mL of appropriate medium was added to the slurry and the tumor pieces were further mechanically teased apart. The resulting suspension was placed into tissue culture flasks and incubated with the appropriate culture medium supplemented with 100 unit/mL penicillin G sodium and 100 µg/mL streptomycin sulfate (Gibco, Grand Island, NY). Medium was changed every two to three days. After three to five passages, the antibiotic supplements were removed and the cells maintained in antibiotic-free medium.

For the SKOV3TP5 cells, fragments of resected tumors were injected SC into mice for *in vivo* passaging. This procedure was repeated five times before cells were returned to culture as described above.

### Cell Culture

All reagents and media for cell culture were obtained from Life Technologies, Inc. (Gaithersburg, MD). C6 cells were cultured in Ham's F-10 supplemented with 5% FBS and 2mM L-glutamine. A375, A431 and EpH4-VEGF1 cells were cultured in DMEM supplemented with 10% FBS and 2 mM L-glutamine. SKOV3TF5 cells were cultured in McCoy's 5A medium supplemented with 10% FBS and 2mM L-glutamine, Calu-6, SF763T and SF767T cells were cultured in MEM supplemented with 10% FBS, 2 mM L-glutamine, 1 mM sodium pyruvate and 0.1 mM MEM non-essential amino acids. All cell cultures were maintained at 37°C in a humidified atmosphere of 5-10% CO₂.

### Subcutaneous Xenograft Models in Athymic Mice

Experiments were conducted in accordance with Institutional Animal Care and Use Committee (IACUC) guidelines as listed under protocol SAF029. Tumor cell lines (0.6 - 10 x 10⁶ cells/animal; n = 8-20 animals/group) were implanted SC in the hindflank region of 8-12 week old BALB/c nu/nu female mice. With the exception of experiments where the efficacy of 3-[2,4-Dimethyl-5-(2-oxo-1,2-dihydro-indol-3-ylidenemethyl)-1H-pyrrol-3-yl]-propionic acid on established tumors was evaluated, treatment with either 3-[2,4-Dimethyl-5-(2-oxo-1,2-dihydro-indol-3-ylidenemethyl)-1H-pyrrol-3-yl]-propionic acid or vehicle commenced one day post-implantation.

For parenteral dosing, animals received a 50 µL IP bolus injection of either 3-[2,4-Dimethyl-5-(2-oxo-1,2-dihydro-indol-3-ylidenemethyl)-1H-pyrrol-3-yl]-propionic acid in DMSO or DMSO alone for the indicated number of days. In most experiments, compound was administered daily. In the dosing regimen studies, compound was administered according to the schedules outlined in Table 2.

For oral administration, 3-[2,4-Dimethyl-5-(2-oxo-1,2-dihydro-indol-3-ylidenemethyl)-1H-pyrrol-3-yl]-propionic acid was delivered by oral gavage (PO) at 200 mg/kg in a Labrasol®-containing vehicle.

Tumor growth was measured using venier calipers. Tumor volumes were calculated as the product of length x width x height. Statistical analysis was carried out using Student's t-test.

### Results

Significant inhibition of SC tumor growth of Calu-6 (human lung carcinoma) cells in BALB/c nu/nu mice was observed following IP administration of 3-[2,4-Dimethyl-5-(2-oxo-1,2-dihydro-indol-3-ylidenemethyl)-1H-pyrrol-3-yl]-propionic acid at 75 and 100 mg/kg/day. At the conclusion of the experiment (Day 25), inhibition of tumor growth was 79 to 86% compared to vehicle-treated control animals, and mortality was 12.5% at both doses.

In subsequent experiments, 3-[2,4-Dimethyl-5-(2-oxo-1,2-dihydro-indol-3-ylidenemethyl)-1H-pyrrol-3-yl]-propionic acid was administered IP to mice which had received implants of tumor xenografts representing several different tissue origins. The results of these studies are summarized in Table 2.

**Table 2**

| Effect of TP administered 3-[2,4-Dimethyl-5-(2-oxo-1,2-dihydro-indol-3-ylidenemethyl)-1H-pyrrol-3-yl]-propionic acid on SC growth of a panel of tumor xenografts in BALB/c nu/nu mice | | | | | | |
|---|---|---|---|---|---|---|
| **Test Facility Number** | **Cell Line** | **Tumor Type** | **Compound Dose (mg/kg/d)** | **% Inhibition @ (day)** | **P value** | **% Mortality** |
| E01591 | A375 | human melanoma | 75* | 56(29) | 0.4386 | 0 |
| E01592 | C6 | rat glioma | 100 | 65 (18) | 0.0004 | 12.5 |
| E01606 | SF763T | human glioma | 75 | 48 (21) | 0.0037 | 12.5 |
| E01607 | SF767T | human glioma | 75 | 46 (21) | 0.0041 | 12.5 |
| E01668 | SKOV3TP5 | human ovarian | 75 | 9 (25) | ns | 25 |
| E01656 | A431 | human epidermal | 100 | 84 (24) | <0.0001 | 12.5 |
| E01661 | EpH4-VEGF1 | murine epithelial | 75 | 34 (18) | 0.0339 | 0 |

| | | | | | | |
|---|---|---|---|---|---|---|
| Table 2. Daily administration (except where indicated by *, dosed 3x/week) at the indicated doses (mg/kg/day) in DMSO began one day post-implantation. Percent inhibition compared to the vehicle-treated control group was calculated on the indicated days post-implantation. P values were calculated by comparing mean tumor size of the treated group to mean tumor size of the vehicle control group using the Student's t-test as = not significant (P > 0.05). | | | | | | |

Including the Calu-6 data depicted in Table 2, 3-[2,4-Dimethyl-5-(2-oxo-1,2-dihydro-indol-3-ylidenemethyl)-1H-pyrrol-3-yl]-propionic acid significantly inhibited the SC growth of 6 to 7 tumor cell lines tested, representing tumors of skin, brain, and lung origin. These results suggest that 3-[2,4-Dimethyl-5-(2-oxo-1,2-dihydro-indol-3-ylidenemethyl)-1H-pyrrol-3-yl]-propionic acid inbibits the *in vivo* growth of multiple tumor types when administered systemically in a daily regimen.

The studies described above demonstrated that 3-[2,4-Dimettlyl-5-(2-oxo-1,2-dihydro-indol-3-ylidenemethyl)-1H-pyrrol-3-yl]-propionic acid was efficacious at inhibiting the growth of several tumor types *in vivo* if drug administration began one day post-implantation. In the clinical scenario, this might simulate therapy on regrowth of tumors following tumor shrinkage by surgical resection or chemotherapy, or following metastastis of cancer.

To evaluate the efficacy of 3-[2,4-Dimethyl-5-(2-oxo-1,2-dihydro-indol-3, ylidenemethyl)-1H-pyrrol-3-yl]-propionic acid as first line therapy, that is, its effect on tumors that are already established, tumors were implanted and allowed to grow to a measurable size (approximately 100 mm³) prior to initiation of drug administration.

C6 cells were implanted SC into athymic mice on day zero. When measurable tumors had developed in a majority of the animals (Day 7), they were divided into 3 groups: Group 1 received daily IP administration of compound (75 mg/kg/day) from Day 7 through Day 16, Group 2 received daily IP administration of compound (75 mg/kg/day) from Day 7 through Day 22, and Group 3 received daily IP administration of DMSO from Day 7 though Day 16. On Day 16, all Group 3 animals were humanely sacrificed due to tumor burden (mean tumor diameter approximately 1300 mm³), and the growth of tumors in Groups 1 and 2 was monitored until the mean tumor burden of each group reached 1300 mm³.

The results demonstrate that 3-[2,4-Dimethyl-5-(2-oxo-1,2-dihydro-indol-3-ylidenemethyl)-1H-pyrrol-3-yl]-propionic acid was efficacious even when the onset of administration was delayed until measurable tumors were present. Furthermore, there was a finite window of efficacy (Days 7 through 21, Group 1) that was not significantly prolonged by continued dosing beyond Day 16 (Group 2).

To assist in the development of a clinical dosing plan, dosing regimen studies were conducted to determine if tumor growth could be inhibited by dosing less frequently than daily. The results are summarized in Table 3. For comparison, the results obtained with daily dosing are included.

**Table 3**

| *In vivo* dosing regimen studies with IP administered 3-[2,4-Dimethyl-5-(2-oxo-1,2-dihydro-indol-3-ylidenemethyl)-1H-pyrrol-3-yl]-propionic acid | | | | |
|---|---|---|---|---|
| **Test Facility Number** | **Dose (mg/kg)** | **Dosing Regimen** | **% Inhibition (at day).** | **P Value** |
| E01592 | 50 | Daily | 27 (18) | ns |
| E01592 | 75 | Daily | 53 (18) | 0.002 |
| E01592 | 100 | Daily | 65 (18) | 0.004 |
| E01590 | 25 | Daily X 5 | 1(18) | ns |
| E01590 | 50 | Daily X 5 | 68 (18) | 0.0000 |
| E01590 | 75 | Daily X 5 | 54 (18) | 0.0004 |
| E01590 | 50 | Twice weekly | 36 (19) | 0.0175 |
| E01590 | 75 | Twice weekly | 26 (19) | ns |
| E01590 | 100 | Twice weekly | 38 (19) | 0.0188 |
| E01590 | 50 | Three times weekly | 25 (18) | ns |
| E01590 | 75 | Three times weekly | 29 (18) | ns |
| E01590 | 100 | Three times weekly | 40 (18) | 0.0042 |
| E01605 | 100 | Daily X 5 followed by Once weekly | 42(15) | 0.0327 |
| E01605 | 150 | Daily X 5 followed by once weekly | 51 (15) | 0.0128 |

| | | | | |
|---|---|---|---|---|
| Table 3. C6 cells were implanted SC in BALB/c nu/nu mice. Beginning one day post-implantation, animals received the indicated doses of compound, administered IP according to the schedules indicated. Control animals were dosed with vehicle (DMSO) according to the same schedules. Tumor volumes were measured and percent inhibition was calculated as described in the legend to Figure 1. n = 8 - 16 animals/group. ns =not significant (P > 0.05). | | | | |

These data suggest that 3-[2,4-Dimethyl-5-(2-oxo-1,2-dihydro-indol-3-ylidenemethyl)-1H-pyrrol-3-yl]-propionic acid can be delivered using a dosing regimen that is less frequent than daily administration. For example, doses of 50 to 75 mg/kg delivered Daily X 5 yielded efficacy similar to that seen when 100 mg/kg was administered Daily X 7. Also, a dose of 100 mg/kg was administered twice weekly or three times weekly yielded statistically significant tumor growth inhibition of approximately 40%. Thus, daily administration of 3-[2,4-Dimethyl-5-(2-oxo-1,2-dihydro-indol-3-ylidenemethyl)-1H-pyrrol-3-yl]-propionic acid is not necessary to achieve significant efficacy in preclinical models.

The efficacy of 3-[2,4-Dimethyl-5-(2-oxo-1,2-dihydro-indol-3-ylidenemethyl)-1H-pyrrol-3-yl]-propionic acid by the oral route of administration was examined in the C6 (rat glioma) SC xenograft model in athymic mice. Beginning one day post-implantation, compound (200 mg/kg/day in solution in a Labrasol®-containing vehicle) was administered to mice PO. Tumor growth was measured using venier calipers and tumor volumes were calculated as the product of length x width x height.

In this SC xenograft model, significant inhibition of tumor growth occurred following oral treatment of mice with 3-[2,4-Dimethyl-5-(2-oxo-1,2-dihydro-indol-3-ylidenemethyl)-1H-pyrrol-3-yl]-propionic acid at 200 mg/kg/day. On Day 18 post-implantation, tumor growth was inhibited by 82% compared to vehicle-treated animals, with only 10% mortality. These data demonstrate that 3-[2,4-Dimethyl-5-(2-oxo-1,2-dihydro-indol-3-ylidenemelhyl)-1H-pyrrol-3-yl]-propionic acid effectively inhibited tumor growth when administered PO

### EXAMPLE 2: Preformulation Studies

Preformulation studies evaluate the physico-chemical properties of the compound and enable formulation development of the compound.

### Solubility Profile

Solubility was determined at room temperature by shaking an excess amount of 3-[2,4-dimethyl-5-(2-oxo-1,2-dihydro-indol-3-ylidenetnethyl)-1H-pyrrol-3-yl]-propionic acid in solvent for over 24 hours. The solution was filtered, appropriate dilutions made and the concentration of the compound in the supernatant was analyzed by a reverse phase HPLC method. The results are tabulated in Table 4.

**Table 4**

| **Solubility of 3-[2,4-Dimethyl-5-(2-oxo-1,2-dihydro-indol-3-ylidenemethyl)-1H-pyrrol-3-yl]-propionic acid** | |
|---|---|
| **Solvent** | **Solubility (mg/mL)** |
| Water* | 0.03 |
| 0.1N HCl | <0.0001 |
| 0-1N NaOH* | 20.8 |
| pH 6 buffer** | 0.002 |
| pH 7 buffier** | 0.009 |
| pH 8 buffer** | 0.085 |
| PH 9 buffer** | 1.60 |
| PEG-300* | 6.8 |
| 15% PEG 300 in pH 8.2 buffer** | 1.4 |
| 30% PEG 300 in pH 8.2 buffer** | 32 |
| 15% PEG 300,1% Benzyl alcohol in pH 8.2 buffer** | 1.8 |
| 30% PEG 300, 1% Benzyl alcohol in pH 8.2 buffer** | 3.7 |
| 10% Propylene glycol in pH 8.2 buffer** | 0.4 |
| 2% aqueous Polysorbate - 80 | 0.13 |
| Ethanol | 1.2 |
| Methanol | 0.6 |
| 2% Polysorbate-80 | 0-13 |

| | |
|---|---|
| *Average solubility of 3 lots. ** Average of 2 lots. Buffer pH 6-8.2 is 0.1 M phosphate buffer; buffer pH 9 is 0.1 M borate buffer | |

### pH Solubility Profile

The solubility of two lots of 3-[2,4-Dimethyl-5-(2-oxo-1,2-dihydro-indol-3-ylidenemethyl)-1H-pyrrol-3-yl]-propionic acid was determined at pH 6.0, 6.5, 7.0, 7-5 and 8.0 of phosphate buffer and pH 9 of borate buffer. The results are indicated in Table 5 and show that the solubility increases with pH. Ionic strength of the solution and common ion effect can affect solubility.

**Table 5**

| **Solubility of 3-[2,4-Dimethyl-5-(2-oxo-1,2-dihydro-indol-3-ylidenemethyl)-1H-pyrrol-3-yl]-propionic acid as a function of pH** | | |
|---|---|---|
| **pH of Buffer** | **Average pH at Equilibrium** | **Average Solubility (mg/mL)** |
| 6.0 | 6.06 | 0.0024 |
| 6.5 | 6.52 | 0.004 |
| 7.0 | 6.96 | 0.009 |
| 7.4 | 7.39 | 0.019 |
| 8.0 | 8.06 | 0.085 |
| 8.2 | n/t | 0.13 |
| 9.0 | 8.79 | 1.6 |

| | | |
|---|---|---|
| Buffer pH 6-8.2 was 0.1 M phosphate buffer; buffer pH 9 was 0.1 M borate buffer. "pH at equilibrium" was measured after 24 hours of equilibration, just before analysis. n/t = not tested. | | |

### Ionization Constant, pKA, and Partition Coefficient, Log P

3-[2,4-Dimethyl-5-(2-oxo-1,2-dihydro-indol-3-ylidenemethyl)-1H-pyrrol-3-yl]-propionic acid is a hydrophobic ionizable weak acid. The pKa is 5.02. Log P and Log D (at pH 7.4) are 3.85 and 1.5, respectively. pKa, Log P, and Log D values were determined by potentiometric titration at Robertson Microlit Laboratories, New Jersey. pka was calculated using the Seiler method.

**Table 6**

| **Solubility of *In situ* 3-[2,4-dimethyl-5-(2-oxo-1,2-dihydro-indol-3-ylidenemethyl)-1H-pyrrol-3-yl]-propionic acid salt formation** | | |
|---|---|---|
| **Salt** | **Solubility mg/ml** | **pH** |
| Sodium | 23.9 | 8.56 |
| Ammonium | 3.85 | 9.2 |
| Ethylenediamine | 7.1 | 9.3 |
| Choline | 6.5 | 8.2 |
| Meglumine | 7.9 | 9.3 |
| Buffer pH 8 (free acid) | 0.085 | 8.06 |
| Buffer pH 8.8 (free acid) | 1.6 | 8.8 |
| Sodium salt | 17-25 | 8.6-8.9 |

The preformulation studies indicated that the aqueous solubility of the compound could be enhanced about 1000 times by in situ salt formation (e.g., the sodium salt has a solubility of about 25 mg/ml in comparison to the free acid which is about 30 µg/ml). This strategy was used in the formulation development.

### EXAMPLE 3: Parenteral formulations of Ionizable Substituted Indolinone Compounds

To be used as a parenteral formulation, and especially an intravenous formulation, a high aqueous solubility of the drug candidate is desirable. The solubility of 3-[2,4-Dimethyl-5-(2-oxo-1,2-dihydro-indol-3-ylidenemethyl)-1H-pyrrol-3-yl]-propionic acid was enhanced several folds by salt formation. The sodium salt of 3-[2,4-Dimethyl-5-(2-oxo-1,2-dihydro-indol-3-ylidenemethyl)-1H-pyrrol-3-yl]-propionic acid has a solubility of 20 mg/mL compared to the less than 0.1 mg/mL of the free acid. The solubility of 3-[2,4-Dimethyl-5-(2-oxo-1,2-dihydro-indol-3-ylidenemethyl)-1H-pyrrol-3-yl]-propionic acid was either enhanced by using the sodium salt of 3-[2,4-Dimethyl-5-(2-oxo-1,2-dihydro-indol-3-ylidenemethyl)-1H-pyrrol-3-yl]-propionic acid or by *in situ* salt formation using acid equivalents of sodium hydroxide during manufacture of the formulation.

Following salt formation, the formulation was buffered with various solutions, including glutamate, glycine, tris-phosphate and sodium hydroxide solutions, at pH ranging from 8-9 at different buffer strengths (0.01, 0.05, and 0.1 M). Some water miscible cosolvents like PEG-300 in varying amounts (0% to 40%) were used to enhance the solubility and to stabilize the formulations. Addition of other solubilizers, such as Polysorbate 80, was also tested

The composition of an exemplary formulations are given in Table 7, below. The formulation if it does not contain any Cremophor® or Polysorbate-80 should be diluted 1:1 (one part formulation to 1 part IV fluid) with intravenous fluid such as sterile water for injection, containing 0.45% sodium chloride, before administration. The dilution is much higher (1:1 to 1: 50) depending on the concentration of Polyethoxylated 35 Castor Oil (Cremophor® EL) and Polysorbate-80 These parenteral formulations can also be administered orally.

**Table 7**

| **Parenteral Formulation Composition** | | |
|---|---|---|
| **Component** | ****Concentration Percent(w/v)** | |
| | Formula 1 | Formula 2 |
| *3-[2,4-Dimethyl-5-2-oxo-1,2-dihydro-indol-3-ylidenemethyl)-1H-pirrol-3-yl]-propionic acid | 0.05-1.5 | 0.05-5.0 |
| Polyethylene Glycol 300, NF | Most Preferred 30.0% | Most Preferred 45.0% |
| | More preferably 1.0-70.0% | More preferably 1.0-70.0% |
| | Preferably 10-50 | Preferably 25-65% |
| Benzyl Alcohol, USP/NF | Most Preferred 1.0% | Most Preferred 1.0% |
| | More preferably 0-3.0 % | More preferably 0-3.0 % |
| | Preferably 0.9-2.0 % | Preferably 0.9-2.0 % |
| Polyethoxylated 35 castor oil | Most Preferred 0% | Most Preferred 31.5% |
| (Cremophor® EL) or Polysorbate 80 | More preferably 0-50% | More preferably 0-50% |
| | Preferably 0-10% | Preferably 0-31.5% |
| Sodium Phosphate Dibasic, anhydrous, USP/NF | Most Preferred 0.47% | Most Preferred 0% |
| | More preferably 0-1.0% | More preferably 0-1.0% |
| | Preferably 0.3-0.5 % | Preferably 0.3-0.5 % |
| Sodium Phosphate Monobasic, monohydrate, USP/NF | Most Preferred 0.016% | Most Preferred 0% |
| | More preferably 0-0.25 % | More preferably 0-0.25 % |
| | Preferably 0.01-0.02 % | Preferably 0.01-0.02 % |
| Water for Injection, USP | QS to 100% | QS to 1.0 mL |
| pH adjustment by NaoH or HCl | More preferably 7.5-10.0% | More preferably 7.5-10.0% |
| | Preferably 9.0-9.5% | Preferably 9.0-9.5% |

| | | |
|---|---|---|
| **drug conc. equivalent to 12 mg/mL of 3-[2,4-Dimethyl-5-(2-oxo-1,2-dihydro-indol-3-ylidenemethyl)-1H-pyrrol-3-yl]-propionic acid. *When the free acid is used to manufacture the formulation, a 1:1 molar equivalent of sodium hydroxide is added for in situ salt formation. Values in the first level of parenthesis represent concentration ranges according to preferred embodiments of the invention. The second level of parenthesis are the most preferred embodiments of the invention. | | |

### EXAMPLE 4: Parenteral Formulation Procedures

### Batch size 100 mL

Prepare 100 mL, 0.05 M Phosphate buffer, pH 8.0-9.0, preferably pH 8.2 using sodium phosphate monobasic (monohydrate) and sodium phosphate dibasic (anhydrous).
Weigh the sodium salt of 3-[2,4-dimethyl-5-(2-oxo-1,2-dihydro-indol-3-ylidenemethyl)-1H-pyrrol-3-yl]-propionic acid the free. If using the free acid (3-[2,4-dimethyl-5-(2-oxo-1,2-dihydro-indol-3-ylidenemethyl)-1H-pyrrol-3-yl]-propionic acid), add a molar equivalent of sodium hydroxide.
Add 50-60 mL 0-05 M Phosphate buffer.
Stir until dissolved on a magnetic stirrer.
Add PEG-300 to this mixture. If the formulation contains Cremophor or Surfactant, add it after the addition of PEG-300.
Stir until homogenous.
Add Benzyl Alcohol.
Stir until homogenous.
Qs with phosphate bluffer, pH 8-9.0, preferably pH 8.2
Filter through 0.2 µm nylon filter
   (disposable Nalgene filter unit).

### Preparation of 0.05 M Phosphate Buffer pH 8.2:

Prepare 0.05 M sodium phosphate monobasic (monohydrate) solution to give solution 1.
Prepare 0.05 M sodium phosphate dibasic (anhydrous) solution to give solution 2.

Mix solution 1: solution 2 (0.034:1) to give pH 8.2 phosphate buffer. Check pH and adjust to 8.2 with either solution 1 or solution.

### Example 5 - Oral Administration-Oil Suspension

The formulation described below enhance the oral bioaviability of COMPOUND IV in beagle dogs, a preclinical absorption model. At higher doses in fasted conditions, bioavailability studies in beagle dogs indicated that COMPOUND IV coiuld have a low bioavailability due to its poor solubility.

Two oral oil suspension formulations of COMPOUND IV (50mg/gm-formulation F₃ and 250 mg/gm) were developed and evaluated in dogs for oral bioavailability. Both formulations contain 50 mg/gm of Pluronic F-68. The formulation at 50 mg/gm of COMPOUND IV contains 900 mgs of sesame oil and the one that contains 250 mg/gm of COMPOUND IV contains 700 mgs of sesame oil.

Another formulation composed of as high as 750 mg/gm drug load of COMPOUND IV (formulation F₄) was developed using wet granulation. The formulation contains 200 mgs sesame oil. An emulsion of oil in water was prepared with 50 mg/gm PLURONIC™ F68 as emulsion agent and used as granulating liquid.

**Table 8**

| **Example of Composition. (50 mg/gm to 750 mg/gm)** | | |
|---|---|---|
| **Composition** | **Formulation F₃** | **Formulation F₄** |
| COMPOUND IV | 50 mg | 750 mg |
| Pluronic | 50 mg | 50 mg |
| Sesame Oil | 900 mg | 200 nag |

**Table 9**

| | Mean AUC | |
|---|---|---|
| | (fasted) (mcg*min/ml) | |
| | | sd |
| Tablet (wet granulated) | 471 | 478 |
| Oil Suspension (50mg/g) | 919 | 230 |
| Hi load Oil suspension (250mg/g) | 927 | 308 |

AUC refers to the area under the curve of the plasma concentration time curve and represents oral bioavilability (BA). Higher AUC indicates higher BA. Table 9 indicates that the oil suspensions provide higher BA than a conventional wet granulated tablet

### EXAMPLE 6: Development of Preferred Formulations

The following formulation screening experiments identified preferred formulations.

Experiments were designed to screen various granulations of 3-[2,4-Dimethyl-5-(2-oxo-1,2-dihydro-indol-3-ylidenemethyl)-1H-pyrrol-3-yl]-propionic acid made using different proportions of components and the active pharmaceutical ingredient, with respect to their *in vitro* dissolution performance, as capsule dosage form. A total of 16 formulations were manufactured at 10 g scale each. These are listed in Table 10. Each formulation was tested for a period of 45 minutes.

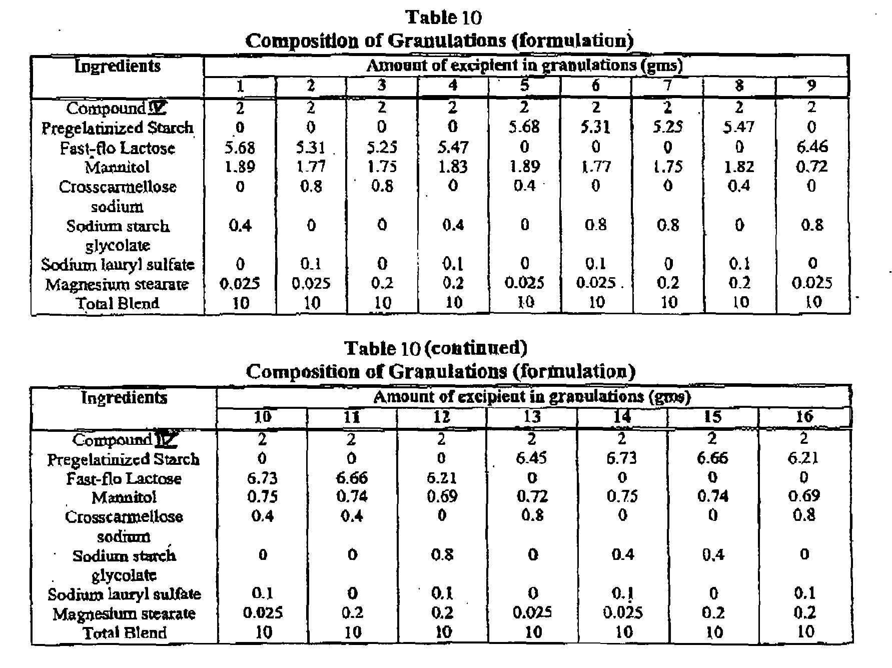

Based on the in-vitro dissolution performance of these granulations, the preferred 4 were identified and these are listed in Table 11. The four preferred dormulations listed in Table 11 were evaluated for stability. These formuations can be encapsulated in different size capsules (e.g., 0, 1, 2, 3 or 4) or tabletted at different strengths.

**Table 11**

| **Clinical Capsule Formulation Candidates** | | | | |
|---|---|---|---|---|
| **Excipients** | **Amout of excipient in granulations (% w/w)** | | | |
| | **F₅ (Dry)** | **F₆ (Dry)** | **F₇ (Dry)** | **F₈ (Wet)** |
| Compound IV⁽¹⁾ | 28.0 | 28.0 | 28.0 | 28.0 |
| Pregelatinized Starch ⁽²⁾ | 0 | 60.0 | 0 | 30.0 |
| Fast-flo Lactose ⁽²⁾ | 65.5 | 0 | 33.5 | 0 |
| Lactose monohydrate regular grade ⁽²⁾ | 0 | 0 | 0 | 33.5 |
| Mannitol⁽²⁾ | 0 | 6.0 | 0 | 0 |
| Microcrystalline cellulose ⁽²⁾ | 0 | 0 | 32.0 | 0 |
| Polyvinylpyroolidone ⁽³⁾ | 0 | 0 | 0 | 2.0 |
| Crosscarmellose sodium ⁽⁴⁾ | 4.0 | 4.0 | 4.0 | 4.0 |
| Sodium lauryl sulfate ⁽⁵⁾ | 1.0 | 1.0 | 0 | 1.0 |
| Cetylpyridinium chloride ⁽⁵⁾ | 0 | 0 | 1.0 | 0 |
| Magnesium stearate ⁽⁶⁾ | 1.0 | 0.5 | 1.0 | 1.0 |
| Colloidal silcon dioxide ⁽⁷⁾ | 0.5 | 0.5 | 0.5 | 0.5 |
| Total Blend | 100 | 100 | 100 | 100 |

| | | | | |
|---|---|---|---|---|
| Note: (1) active ingredient; (2) diluent; (3) binder; (4) disintegrant; (5) wetting agent; (6) lubricant; (7) flow enhancer | | | | |

The data suggest that formulations according to the present invention have improved stability and have increased bioavailability. The *in vivo* data suggest that the formulations are effective in treating PK related disorders such as tumor growth.

## Claims

1. A formulation suitable for parenteral or oral administration, said formulation comprising
(a) an ionizable substituted indolinone, wherein the ionizable substituted indolinone is 3-[2,4-dimethyl-5-(2-oxo-1,2-dihydro-indol-3-ylidenemethyl)-1H-pyrrol-3-yl]-propionic acid, or a pharmaceutically acceptable salt or derivative thereof;
(b) one or more polyoxyhydrocarbyl compounds; and
(c) a pharmaceutically acceptable carrier.

2. The formulation of claim 1, wherein said ionizable substituted indolinone is solubilized by combining said indolinone with a molar equivalent of a base solution or an acid solution.

3. The formulation of claim 1, wherein said formulation is suitable for parenteral administration.

4. The formulation of claim 1, wherein said formulation is suitable for oral administration.

5. The formulation of any one of claims 1-4, wherein each of said one or more polyoxyhydrocarbyl compounds is independently selected from the group consisting of water soluble carbohydrates, water soluble carbohydrate derivatives, water soluble polypeptides, water soluble polymers, water soluble mixed oxyalkylene polymers, the polymeric forms of ethylene glycol, polyethylene glycol 300, polyethylene glycol 400, propyleneglycol, glycerin, and combinations thereof.

6. The formulation of claim 2, wherein said base solution is selected from the group consisting of sodium hydroxide, ammonium hydroxide, triethylamine, ethylenediamine, N-methyl-D-glucamine, choline, and triethanolamine.

7. The formulation of claim 2, wherein said acid solution is selected from the group consisting of hydrochloric acid, sulfuric acid, formic acid, lactic acid, malic acid, succinic acid, acetic acid, methane sulfonic acid, benzene sulfonic acid, and phosphoric acid.

8. The formulation of any one of claims 1-7, wherein said pharmaceutically acceptable carrier further comprises
(a) one or more buffers;
(b) one or more pharmaceutically acceptable surfactants;
(c) one or more pharmaceutically acceptable preservatives;
(d) one or more antioxidants;
(e) one or more pharmaceutically acceptable alcohols;
(f) one or more pharmaceutically acceptable oils;
(g) one or more polyglycolized lipids;
(h) one or more pharmaceutically acceptable granulating agents; or
(i) any combination thereof.

9. The formulation of claim 8, wherein each of said one or more buffers is independently selected from the group consisting of acetate, citrate, phosphoric acid buffer, ascorbate, hydrochloric acid buffer, Tris-HCl buffer, sodium phosphate, sodium carbonate, sodium hydroxide, glutamate, glycine, and Tris base buffers.

10. The formulation of claim 8 or 9, wherein each of said one or more pharmaceutically acceptable surfactants is independently selected from the group consisting of pharmaceutically acceptable non-ionic surfactants and pharmaceutically acceptable anionic surfactants.

11. The formulation of any one of claims 8-10, wherein each of said one or more pharmaceutically acceptable surfactants is a non-ionic surfactant independently selected from the group consisting of polyoxyethylenepolypropylene glycols, polyoxyethylene castor oil derivatives, polyoxyethyleneglycerol oxystearate.

12. The formulation of any one of claims 8-11, wherein each of said one or more pharmaceutically acceptable preservatives is independently selected from the group consisting of benzyl alcohol, methyl paraben, ethyl paraben, and phenol.

13. The formulation of any one of claims 8-12, wherein each of said one or more antioxidants is independently selected from the group consisting of sodium meta-bisulfite, EDTA, ascorbic acid, and benzyl alcohol.

14. The formulation of any one of claims 8-13, wherein each of said one or more pharmaceutically acceptable alcohols is independently selected from the group consisting of ethanol, benzyl alcohol, propylene glycol, 2-(2-ethoxyethoxy)ethanol, and glycerol.

15. The formulation of any one of claims 8-14, wherein the pharmaceutically acceptable carrier further comprises one or more pharmaceutically acceptable alcohols, and wherein the pharmaceutically acceptable carrier further comprises an amount of pharmaceutically acceptable aqueous solution effective to prevent hemolysis on parenteral administration to a patient in need thereof.

16. The formulation of any one of claims 8-15, wherein each of said one or more pharmaceutically acceptable oils is independently selected from the group consisting of mineral oils, vegetable oils, fractionated coconut oils, sesame oil, propyleneglycol monolaurate, and mixed triglycerides with caprylic acid and capric acid.

17. The formulation of any one of claims 8-16, wherein each of said one or more polyglycolized lipids is independently selected from the group consisting of monoglycerides, diglycerides, triglycerides, polyethyleneglycol monoesters, and polyethyleneglycol diesters.

18. The formulation of any one of claims 8-17, wherein each of said pharmaceutically acceptable granulating agents is selected from the group consisting of silicon dioxide, microcrystalline cellulose, starch, calcium carbonate, pectin, crospovidone, water, alcohol and polyplasdone or a combination of any of the preceeding.

19. The formulation of claim 4, wherein said pharmaceutically acceptable carrier comprises two or more of the members of the group consisting of one or more polyoxyhydrocarbyl compounds, one or more polyglycolized lipids, one or more sufactants, and one or more granulizing agents.

20. The formulation of claim 19, wherein said pharmaceutically acceptable carrier comprises one or more polyoxyhydrocarbyl compounds, one or more polyglycolized lipids, and one or more surfactants.

21. The formulation of claim 4, wherein said formulation is solid, and wherein said pharmaceutically acceptable carriers comprise one or more pharmaceutically acceptable diluents, one or more pharmaceutically acceptable binders, one or more pharmaceutically acceptable disintegrants, one or more pharmaceutically acceptable surfactants, one or more pharmaceutically acceptable lubricants, and one or more pharmaceutically acceptable flow enhancers.

22. The formulation of claim 21, wherein
(a) each of said one or more pharmaceutically acceptable diluents is selected from the group consisting of pregelatinized starch, lactose monohydrate, lactose, monohydrate regular grade, mannitol, calcium phosphate and microcrystalline cellulose;
(b) each of said one or more pharmaceutically acceptable binders is selected from the group consisting of polyvinylpyrrolidone, hydroxypropylmethyl cellulose, hydroxypropylcellulose and starch;
(c) each of said one or more pharmaceutically acceptable disintegrants is selected from the group consisting of crosscarmellose sdoium, sodium starch glycolate, gospovidone, and starch;
(d) each of said one or more pharmaceutically acceptable surfactants is selected from the group consisting of sodium lauryl sulfate, polysorbate and cetylpyridinium chloride;
(e) each of said one or more pharmaceutically acceptable lubricants is selected from the group consisting of magnesium stearate, sodium stearyl fumarate, glyceryl behenate and stearic acid; or
(f) each of said one or more pharmaceutically acceptable flow enhancers is selected from the group consisting of colloidal silicon dioxide and talc.

23. The formulation of claim 4, wherein said formulation is a solution, and wherein
(a) said pharmaceutically acceptable carrier comprises one or more polyoxyhydrocarbyl compounds, one or more surfactants, and one or more buffers;
(b) the polyoxyhydrocarbyl compound is selected from the group consisting of water soluble carbohydrates, water soluble carbohydrate derivatives, water soluble polypeptides, water soluble polymers, water soluble mixed oxyalkylene polymers, the polymeric forms of ethylene glycol, and combinations thereof;
(c) the surfactant is selected from the group consisting of pharmaceutically acceptable non-ionic surfactants and pharmaceutically acceptable anionic surfactants; or
(d) the buffer is selected from the group consisting of acetate, citrate, phosphoric acid buffer, ascorbate, hydrochloric acid buffer, Tris-HCl buffer, sodium phosphate, sodium carbonate, sodium hydroxide, glutamate, glycine, and Tris base buffers.

24. The formulation of claim 4, wherein said formulation is an aqueous suspension, and wherein
(a) the pharmaceutically acceptable carrier comprises a suspending agent and a surfactant;
(b) the suspending agent is selected from the group consisting of carboxymethylcellulose, hydroxypropylmethylcellulose, povidone and starch; or
(c) the surfactant is selected from the group consisting of pharmaceutically acceptable non-ionic surfactants and pharmaceutically acceptable anionic surfactants.

25. The formulation of claim 3, wherein
(a) pharmaceutically acceptable carrier comprises one or more pharmaceutically acceptable surfactants and one or more pharmaceutically acceptable oils;
(b) the pharmaceutically acceptable surfactant comprises an ethylene oxide copolymer and said pharmaceutically acceptable oil comprises sesame oil;
(c) the ionizable substituted indolinone is present in a concentration selected from the range of about 50 mg/gm to about 750 mg/gm;
(d) the ionizable substituted indolinone is present in a concentration selected from the range of about 50 mg/gm to about 500 mg/gm; or
(e) the ionizable substituted indolinone is present in a concentration selected from the range of about 50 mg/gm to about 200 mg/gm.

26. The formulation of any one of claims 1-25 formulated into a hard gelatin capsule or a soft gelatin capsule.

27. Use of a formulation according to any one of claims 1-26 for the manufacture of a medicament.

28. The use of claim 27, wherein the medicament is useful in preventing or treating a protein kinase related disorder, wherein the protein kinase related disorder is disorder selected from the group consisting of: (a) a cancer which is selected from the group consisting of squamous cell carcinoma, a sarcoma, Kaposi's sarcoma, astrocytoma, glioblastoma, lung cancer, bladder cancer, colorectal cancer, gastrointestinal cancer, head and neck cancer, melanoma, ovarian cancer, prostate cancer, breast cancer, small-cell lung cancer and glioma; (b) diabetes; (c) a hyper-proliferation disorder; (d) von Hippel-Lindau disease; (e) restenosis; (f) fibrosis; (g) psoriasis; (h) osteoarthritis; (i) rheumatoid arthritis; (j) an inflammatory disorder; (k) angiogenesis; (l) an immunological disorder selected from the group consisting of autoimmune disease and AIDS; and (m) a cardiovascular disorder.

29. The use of claim 27 or 28, wherein the ionizable substituted indolinone is present in the medicament at
(a) a concentration in the range from about 50 mg/gm to about 750 mg/gm;
(b) a concentration in the range from about 50 mg/gm to about 500 mg/gm; or
(c) a concentration in the range from about 50 mg/gm to about 250 mg/gm.

30. A method of preparing a formulation comprising:
(a) adding to a salt solution, formed *in situ* by admixing a molar equivalent of a base solution or an acid solution with an ionizable substituted indolinone, wherein said ionizable substituted indolinone is 3-[2,4-dimethyl-5-(2-oxo-1,2-dihydro-indol-3-ylidenemethyl)-1H-pyrrol-3-yl]-propionic acid, or a pharmaceutically acceptable salt or derivative thereof; and
(b) mixing the solution of the ionized substituted indolinone with one or more polyoxyhydrocarbyl compounds and/or one or more buffers.

31. A method of making a formulation suitable for oral administration comprising admixing
(a) an ionizable substituted indolinone, wherein said ionizable substituted indolinone is 3-[2,4-dimethyl-5-(2-oxo-1,2-dihydro-indol-3-ylidenemethyl)-1H-pyrrol-3-yl]-propionic acid, or a pharmaceutically acceptable salt or derivative thereof;
(b) one or more pharmaceutically acceptable surfactants; and
(c) one or more pharmaceutically acceptable oils.

## Patentansprüche

1. Formulierung, geeignet zur parenteralen oder oralen Verabreichung, wobei die Formulierung umfasst:
(a) ein ionisierbares substituiertes Indolinon, wobei das ionisierbare substituierte Indolinon 3-[2,4-Dimethyl-5-(2-oxo-1,2-dihydro-indol-3-ylidenmethyl)-1H-pyrrol-3-yl]-propionsäure ist, oder ein pharmazeutisch verträgliches Salz oder Derivat davon;
(b) eine oder mehrere Polyoxyhydrocarbylverbindung(en); und
(c) einen pharmazeutisch verträglichen Träger.

2. Formulierung gemäß Anspruch 1, wobei das ionisierbare substituierte Indolinon löslich gemacht ist durch Vereinigen des Indolinons mit einem molaren Äquivalent einer Basenlösung oder einer Säurelösung.

3. Formulierung gemäß Anspruch 1, wobei die Formulierung für die parenterale Verabreichung geeignet ist.

4. Formulierung gemäß Anspruch 1, wobei die Formulierung für die orale Verabreichung geeignet ist.

5. Formulierung gemäß einem beliebigen der Ansprüche 1-4, wobei jede der einen oder mehreren Polyoxyhydrocarbylverbindung(en) unabhängig ausgewählt ist aus der Gruppe, bestehend aus wasserlöslichen Kohlehydraten, wasserlöslichen Kohlehydratderivaten, wasserlöslichen Polypeptiden, wasserlöslichen Polymeren, wasserlöslichen gemischten Oxyalkylenpolymeren, den polymeren Formen von Ethylenglykol, Polyethylenglykol 300, Polyethylenglykol 400, Propylenglykol, Glycerin und Kombinationen davon.

6. Formulierung gemäß Anspruch 2, wobei die Basenlösung ausgewählt ist aus der Gruppe, bestehend aus Natriumhydroxid, Ammoniumhydroxid, Triethylamin, Ethylendiamin, N-methyl-D-glucamin, Cholin und Triethanolamin.

7. Formulierung gemäß Anspruch 2, wobei die Säurelösung ausgewählt ist aus der Gruppe, bestehend aus Salzsäure, Schwefelsäure, Ameisensäure, Milchsäure, Äpfelsäure, Bernsteinsäure, Essigsäure, Methansulfonsäure, Benzolsulfonsäure und Phosphorsäure.

8. Formulierung gemäß einem beliebigen der Ansprüche 1-7, wobei der pharmazeutisch verträgliche Träger des Weiteren umfasst
(a) einen oder mehrere Puffer;
(b) eines oder mehrere pharmazeutisch verträgliche Sufactant(s);
(c) einen oder mehrere pharmazeutisch verträgliche Konservierungsstoff(e);
(d) ein oder mehrere Antioxidantien;
(e) einen oder mehrere pharmazeutisch verträgliche(n) Alkohol(e);
(f) ein oder mehrere pharmazeutisch verträgliche(s) Öl (e) ;
(g) ein oder mehrere polyglukolisierte(s) Lipid(e);
(h) ein oder mehrere pharmazeutisch verträgliche(s) Granulierungsmittel; oder
(i) eine beliebige Kombination daraus.

9. Formulierung gemäß Anspruch 8, wobei jeder der einen oder mehreren Puffer unabhängig ausgewählt ist aus der Gruppe, bestehend aus Acetat-, Citrat-, Phosphorsäurepuffer, Ascorbat-, Salzsäurepuffer, Tris-HCl-Puffer, Natriumphosphat-, Natriumcarbonat-, Natriumhydroxid-, Glutamat-, Glycin- und Tris-Base-Puffer.

10. Formulierung gemäß Anspruch 8 oder 9, wobei jedes der einen oder mehreren pharmazeutisch verträglichen Surfactants unabhängig ausgewählt ist aus der Gruppe, bestehend aus pharmazeutisch verträglichen nichtionischen Surfactants und pharmazeutisch verträglichen anionischen Surfactants.

11. Formulierung gemäß einem beliebigen der Ansprüche 8-10, wobei jedes der einen oder mehreren pharmazeutisch verträglichen Surfactants ein nicht ionisches Surfactant ist, unabhängig ausgewählt aus der Gruppe, bestehend aus Polyoxyethylenpolypropylenglykolen, Polyoxyethylencastorölderivaten, Polyoxyethylenglycerinoxystearat.

12. Formulierung gemäß einem beliebigen der Ansprüche 8-11, wobei jeder der einen oder mehreren pharmazeutisch verträglichen Konservierungsstoff(e) unabhängig ausgewählt ist aus der Gruppe, bestehend aus Benzylalkohol, Methylparaben, Ethylparaben und Phenol.

13. Formulierung gemäß einem beliebigen der Ansprüche 8-12, wobei jedes der einen oder mehreren Antioxidantien unabhängig ausgewählt ist aus der Gruppe, bestehend aus Natriummetabisulfit, EDTA, Ascorbinsäure und Benzylalkohol.

14. Formulierung gemäß einem beliebigen der Ansprüche 8-13, wobei jeder der einen oder mehreren pharmazeutisch verträglichen Alkohol(e) unabhängig ausgewählt ist aus der Gruppe, bestehend aus Ethanol, Benzylalkohol, Propylenglykol, 2-(2-Ethoxyethoxy)ethanol und Glycerin.

15. Formulierung gemäß einem beliebigen der Ansprüche 8-14, wobei der pharmazeutisch verträgliche Träger des Weiteren einen oder mehrere pharmazeutisch verträgliche(n) Alkohol(e) umfasst, und wobei der pharmazeutisch verträgliche Träger des Weiteren eine Menge pharmazeutisch verträgliche wässrige Lösung umfasst, die wirksam der Hämolyse bei parenteraler Verabreichung an einen Patienten, der Bedarf daran hat, vorbeugt.

16. Formulierung gemäß einem beliebigen der Ansprüche 8-15, wobei jedes der einen oder mehreren pharmazeutisch verträglichen Öle unabhängig ausgewählt ist aus der Gruppe, bestehend aus Mineralölen, pflanzlichen Ölen, fraktionierten Kokosnussölen, Sesamöl, Propylenglycolmonolaurat und gemischten Triglyceriden mit Caprylsäure und Caprinsäure.

17. Formulierung gemäß einem beliebigen der Ansprüche 8-16, wobei jedes der einen oder mehreren polyglykolisierten Lipid(e) unabhängig ausgewählt ist aus der Gruppe, bestehend aus Monoglyceriden, Diglyceriden, Triglyceriden, Polyethylenglycolmonoestern und Polyethylenglycoldiestern.

18. Formulierung gemäß einem beliebigen der Ansprüche 8-17, wobei jedes der pharmazeutisch verträglichen Granulierungsmittel ausgewählt ist aus der Gruppe, bestehend aus Siliciumdioxid, mikrokristalliner Zellulose, Stärke, Calciumcarbonat, Pektin, Crospovidon, Wasser, Alkohol und Polyplasdon oder einer Kombination von beliebigen der zuvor genannten.

19. Formulierung gemäß Anspruch 4, wobei der pharmazeutisch verträgliche Träger zwei oder mehr der Mitglieder der Gruppe umfasst, bestehend aus einer oder mehreren Polyoxyhydrocarbylverbindung(en), einem oder mehreren polyglykolisierten Lipid(en), einem oder mehreren Surfactants(s), und einem oder mehreren Granulierungsmittel(n).

20. Formulierung gemäß Anspruch 19, wobei der pharmazeutisch verträgliche Träger eine oder mehrere Polyoxyhydrocarbylverbindung(en) umfasst, eines oder mehrere polyglykolisierte Lipid(e) und eines oder mehrere Surfactant(s).

21. Formulierung gemäß Anspruch 4, wobei die Formulierung fest ist, und wobei die pharmazeutisch verträglichen Träger ein oder mehrere pharmazeutisch verträgliche Verdünnungsmittel, ein oder mehrere pharmazeutisch verträgliche Bindemittel, ein oder mehrere pharmazeutisch verträgliche(s) Sprengmittel, ein oder mehrere pharmazeutisch verträgliche Surfactant(s), ein oder mehrere pharmazeutisch verträgliche(s) Schmiermittel, und ein oder mehrere pharmazeutisch verträgliche(s) Fließmittel umfassen.

22. Formulierung gemäß Anspruch 21, wobei
(a) jedes der einen oder mehreren pharmazeutisch verträglichen Verdünnungsmittel ausgewählt ist aus der Gruppe, bestehend aus vorgelatinierter Stärke, Laktosemonohydrat, Laktosemonohydrat gewöhnlicher Reinheit, Mannitol, Calciumphosphat und mikrokristalliner Cellulose;
(b) jedes der genannten einen oder mehreren pharmazeutisch verträglichen Bindemittel ausgewählt ist aus der Gruppe, bestehend aus Polyvinylpyrrolidon, Hydroxypropylmethylcellulose, Hydroxypropylcellulose und Stärke;
(c) jedes der genannten einen oder mehreren pharmazeutisch verträglichen Sprengmittel ausgewählt ist aus der Gruppe, bestehend aus Crosscarmellosenatrium, Natriumstärkeglycolat, Gospovidon und Stärke;
(d) jedes der genannten einen oder mehreren genannten pharmazeutisch verträglichen Surfactant(s) ausgewählt ist aus der Gruppe, bestehend aus Natriumlaurylsulfat, Polysorbat und Cetylpyridiniumchlorid;
(e) jedes der genannten einen oder mehreren pharmazeutisch verträglichen Schmiermittel ausgewählt ist aus der Gruppe, bestehend aus Magnesiumstearat, Natriumstearylfumarat, Glycerylbehenat und Stearinsäure; oder
(f) jedes der genannten einen oder mehreren pharmazeutisch verträglichen Fließmittel ausgewählt ist aus der Gruppe, bestehend aus kolloidalem Siliciumdioxid und Talkum.

23. Formulierung gemäß Anspruch 4, wobei die Formulierung eine Lösung ist, und wobei
(a) der pharmazeutisch verträgliche Träger eine oder mehrere Polyoxyhydrocarbylverbindung(en) umfasst, ein oder mehrere Tensid(e) und einen oder mehrere Puffer;
(b) die Polyoxyhydrocarbylverbindung ist ausgewählt aus der Gruppe, bestehend aus wasserlöslichen Kohlehydraten, wasserlöslichen Kohlehydratderivaten, wasserlöslichen Polypeptiden, wasserlöslichen Polymeren, wasserlöslichen gemischten Oxyalkylenpolymeren, den polmeren Formen von Ethylenglykol, und Kombinationen davon;
(c) das Surfactant ist ausgewählt aus der Gruppe, bestehend aus pharmazeutisch verträglichen nichtionischen Surfactants und pharmazeutisch verträglichen anionischen Surfactants; oder
(d) der Puffer ist ausgewählt aus der Gruppe, bestehend aus Acetat-, Citrat-, Phosphorsäurepuffer, Ascorbat-, Salzsäurepuffer, Tris-HCl-Puffer, Natriumphosphat-, Natriumcarbonat-, Natriumhydroxid-, Glutamat-, Glycin- und Tris-Base-Puffer.

24. Formulierung gemäß Anspruch 4, wobei die Formulierung eine wässrige Suspension ist und wobei
(a) der pharmazeutisch verträgliche Träger ein Suspendierungsmittel und ein Surfactant umfasst;
(b) das Suspendierungsmittel ausgewählt ist aus der Gruppe, bestehend aus Carboxymethylcellulose, Hydroxypropylmethylcellulose, Povidon und Stärke; oder
(c) das Surfactant ausgewählt ist aus der Gruppe, bestehend aus pharmazeutisch verträglichen nichtionischen Surfactants und pharmazeutisch verträglichen anionischen Surfactants.

25. Formulierung gemäß Anspruch 3, wobei
(a) der pharmazeutisch verträgliche Träger ein oder mehrere pharmazeutisch verträgliche Surfactant(s) und ein oder mehrere pharmazeutisch verträgliche Öl(e) umfasst;
(b) das pharmazeutisch verträgliche Surfactant ein Ethylenoxidcopolymer umfasst und das pharmazeutisch verträgliche Öl Sesamöl umfasst;
(c) das ionisierbare substituierte Indolinon in einer Konzentration vorhanden ist, ausgewählt aus dem Bereich von etwa 50 mg/g bis etwa 750 mg/g;
(d) das ionisierbare substituierte Indolinon in einer Konzentration vorhanden ist, ausgewählt aus dem Bereich von etwa 50 mg/g bis etwa 500 mg/g;
(e) das ionisierbare substituierte Indolinon in einer Konzentration vorhanden ist, ausgewählt aus dem Bereich von etwa 50 mg/g bis etwa 200 mg/g.

26. Formulierung gemäß einem beliebigen der Ansprüche 1-25, formuliert zu einer Hartgelatinekapsel oder Weichgelatinekapsel.

27. Verwendung einer Formulierung gemäß einem beliebigen der Ansprüche 1-26 zur Herstellung eines Medikaments.

28. Verwendung gemäß Anspruch 27, wobei das Medikament verwendbar ist zur Vorbeugung oder Behandlung einer proteinkinasebedingten Störung, wobei die proteinkinasebedingte Störung eine Störung ist, ausgewählt aus der Gruppe, bestehend aus: (a) einer Krebsart, die ausgewählt ist aus der Gruppe, bestehend aus squamösem Zellkarzinom, einem Sarkom, Kaposi-Sarkom, Astrozytom, Glioblastom, Lungenkrebs, Blasenkrebs, Dickdarmkrebs, Gastrointestinalkrebs, Kopf- und Halskrebs, Melanom, Eierstockkrebs, Prostatakrebs, Brustkrebs, kleinzelligem Lungenkrebs und Geliom; (b) Diabetes; (c) einer Hyperproliferationsstörung; (d) von Hippel-Lindau-Erkrankung; (e) Restenose; (f) Fibrose; (g) Psoriasis; (h) Osteoarthritis; (i) rheumatoider Arthritis; (j) einer Entzündungserkrankung; (k) Angiogenese; (l) einer immunologischen Störung, ausgewählt aus der Gruppe, bestehend aus Autoimmunkrankheit und AIDS; und (m) einer kardiovaskulären Störung.

29. Verwendung gemäß Anspruch 27 oder 28, wobei das ionisierbare substituierte Indolinon in dem Medikament vorhanden ist in
(a) einer Konzentration im Bereich von etwa 50 mg/g bis etwa 750 mg/g;
(b) einer Konzentration im Bereich von etwa 50 mg/g bis etwa 500 mg/g;
(c) einer Konzentration im Bereich von etwa 50 mg/g bis etwa 250 mg/g;

30. Verfahren zur Herstellung einer Formulierung, umfassend:
(a) Zugabe zu einer Salzlösung, gebildet in situ durch Vermischen des molaren Äquivalents einer Basenlösung oder einer Säurelösung mit einem ionisierbaren substituierten Indolinon, wobei das ionisierbare substituierte Indolinon 3-[2,4-Dimethyl-5-(2-oxo-1,2-dihydro-indol-3-ylidenmethyl)-1H-pyrrol-3-yl]-propionsäure ist, oder ein pharmazeutisch verträgliches Salz oder Derivat davon; und
(b) Mischen der Lösung des ionisierten substituierten Indolinons mit einer oder mehreren Polyoxyhydrocarbylverbindung(en) und/oder einem oder mehreren Puffer(n).

31. Verfahren zu Herstellung einer für orale Verabreichung geeigneten Formulierung, umfassend Mischen von:
(a) einem ionisierbaren substituierten Indolinon, wobei das ionisierbare substituierte Indolinon 3-[2,4-Dimethyl-5-(2-oxo-1,2-dihydro-indol-3-ylidenmethyl)-1H-pyrrol-3-yl]-propionsäure ist, oder ein pharmazeutisch verträgliches Salz oder Derivat davon;
(b) einem oder mehreren pharmazeutisch verträglichen Surfactant(s);
(c) einem oder mehreren pharmazeutisch verträglichen Öl(en).

## Revendications

1. Formulation convenant à l'administration parentérale ou orale, ladite formulation comprenant
(a) une indolidone substituée ionisable, ladite indolidone substituée ionisable étant l'acide 3-[2,4-diméthyl-5-(2-oxo-1,2-dihydro-indol-3-ylidèneméthyl)-1H-pyrrol-3-yl]-propionique ou un sel pharmaceutiquement acceptable ou un dérivé de celle-ci ;
(b) un ou plusieurs composés polyoxyhydrocarbyle ; et
(c) un support pharmaceutiquement acceptable.

2. Formulation selon la revendication 1, dans laquelle ladite indolidone substituée ionisable est solubilisée par combinaison de ladite indolidone avec un équivalent molaire d'une solution basique ou d'une solution acide.

3. Formulation selon la revendication 1, dans laquelle ladite formulation convient à l'administration parentérale.

4. Formulation selon la revendication 1, dans laquelle ladite formulation convient à l'administration orale.

5. Formulation selon l'une quelconque des revendications 1-4, dans laquelle chacun desdits un ou plusieurs composés polyoxyhydrocarbyle est indépendamment sélectionné dans le groupe consistant en les glucides hydrosolubles, les dérivés de glucides hydrosolubles, les polypeptides hydrosolubles, les polymères hydrosolubles, les polymères oxyalkylène mixtes hydrosolubles, les formes polymères de l'éthylène glycol, le polyéthylène glycol 300, le polyéthylène glycol 400, le propylèneglycol, la glycérine, et les combinaisons de ceux-ci.

6. Formulation selon la revendication 2, dans laquelle ladite solution basique est sélectionnée dans le groupe consistant en l'hydroxyde de sodium, l'hydroxyde d'ammonium, la triéthylamine, l'éthylènediamine, la N-méthyl-D-glucamine, la choline et la triéthanolamine.

7. Formulation selon la revendication 2, dans laquelle ladite solution acide est sélectionnée dans le groupe consistant en l'acide chlorhydrique, l'acide sulfurique, l'acide formique, l'acide lactique, l'acide malique, l'acide succinique, l'acide acétique, l'acide méthane sulfonique, l'acide benzène sulfonique, et l'acide phosphorique.

8. Formulation selon l'une quelconque des revendications 1-7, dans laquelle ledit support pharmaceutiquement acceptable comprend en outre :
(a) un ou plusieurs tampons ;
(b) un ou plusieurs tensioactifs pharmaceutiquement acceptables ;
(c) un ou plusieurs conservateurs pharmaceutiquement acceptables ;
(d) un ou plusieurs antioxydants ;
(e) un ou plusieurs alcools pharmaceutiquement acceptables ;
(f) une ou plusieurs huiles pharmaceutiquement acceptables ;
(g) un ou plusieurs lipides polyglycolysés ;
(h) un ou plusieurs agents de granulation pharmaceutiquement acceptables ; ou
(i) toutes combinaisons de ceux-ci.

9. Formulation selon la revendication 8, dans laquelle chacun desdits un ou plusieurs tampons est indépendamment sélectionné dans le groupe consistant en les tampons à base d'acétate, de citrate, d'acide phosphorique, d'ascorbate, d'acide chlorhydrique, de Tris-HCl, de phosphate de sodium, de carbonate de sodium, d'hydroxyde de sodium, de glutamate, de glycine, et de Tris-base.

10. Formulation selon la revendication 8 ou 9, dans laquelle chacun desdits un ou plusieurs tensioactifs pharmaceutiquement acceptables est indépendamment sélectionné dans le groupe consistant en les tensioactifs non-ioniques pharmaceutiquement acceptables et les tensioactifs anioniques pharmaceutiquement acceptables.

11. Formulation selon l'une quelconque des revendications 8-10, dans laquelle chacun desdits un ou plusieurs tensioactifs pharmaceutiquement acceptables est un tensioactif non-ionique indépendamment sélectionné dans le groupe consistant en les polyoxyéthylènepolypropylène glycols, les dérivés polyoxyéthylène-huile de ricin, et le polyoxyéthylèneglycérol oxystéarate.

12. Formulation selon l'une quelconque des revendications 8-11, dans laquelle chacun desdits un ou plusieurs conservateurs pharmaceutiquement acceptables est indépendamment sélectionné dans le groupe consistant en l'alcool benzylique, le méthyl paraben, l'éthyl paraben et le phénol.

13. Formulation selon l'une quelconque des revendications 8-12, dans laquelle chacun desdits un ou plusieurs antioxydants est indépendamment sélectionné dans le groupe consistant en le métabisulfite de sodium, l'EDTA, l'acide ascorbique et l'alcool benzylique.

14. Formulation selon l'une quelconque des revendications 8-13, dans laquelle chacun desdits un ou plusieurs alcools pharmaceutiquement acceptables est indépendamment sélectionné dans le groupe consistant en l'éthanol, l'alcool benzylique, le propylène glycol, le 2-(2-éthoxyéthoxy)éthanol et le glycérol.

15. Formulation selon l'une quelconque des revendications 8-14, dans laquelle le support pharmaceutiquement acceptable comprend un ou plusieurs alcools pharmaceutiquement acceptables et dans laquelle ledit support pharmaceutiquement acceptable comprend, en outre, une quantité d'une solution aqueuse pharmaceutiquement acceptable efficace à prévenir l'hémolyse lors de l'administration parentérale à un patient nécessitant une telle administration.

16. Formulation selon l'une quelconque des revendications 8-15, dans laquelle chacune desdites une ou plusieurs huiles pharmaceutiquement acceptables est indépendamment sélectionnée dans le groupe consistant en les huiles minérales, les huiles végétales, les huiles de coprah fractionnées, l'huile de sésame, le monolaurate de propylèneglycol, et les triglycérides mixtes avec l'acide caprylique et l'acide caprique.

17. Formulation selon l'une quelconque des revendications 8-16, dans laquelle chacun desdits un ou plusieurs lipides polyglycolysés est indépendamment sélectionné dans le groupe consistant en les monoglycérides, les diglycérides, les triglycérides, les monoesters du polyéthylèneglycol et les diesters du polyéthylèneglycol.

18. Formulation selon l'une quelconque des revendications 8-17, dans laquelle chacun desdits agents de granulation pharmaceutiquement acceptables est sélectionné dans le groupe consistant en le dioxyde de silicium, la cellulose microcristalline, l'amidon, le carbonate de calcium, la pectine, la crospovidone, l'eau, l'alcool et la polyplasdone ou une combinaison quelconque de ceux-ci.

19. Formulation selon la revendication 4, dans laquelle ledit support pharmaceutiquement acceptable comprend deux, ou davantage, membres du groupe consistant en un ou plusieurs composés polyoxyhydrocarbyle, un ou plusieurs lipides polyglycolysés, un ou plusieurs tensioactifs et un ou plusieurs agents de granulation.

20. Formulation selon la revendication 19, dans laquelle ledit support pharmaceutiquement acceptable comprend un ou plusieurs composés polyoxyhydrocarbyle, un ou plusieurs lipides polyglycolysés et un ou plusieurs tensioactifs.

21. Formulation selon la revendication 4, dans laquelle ladite formulation est solide et dans laquelle lesdits supports pharmaceutiquement acceptables comprennent un ou plusieurs diluants pharmaceutiquement acceptables, un ou plusieurs liants pharmaceutiquement acceptables, un ou plusieurs délitants pharmaceutiquement acceptables, un ou plusieurs tensioactifs pharmaceutiquement acceptables, un ou plusieurs lubrifiants pharmaceutiquement acceptables et un ou plusieurs améliorateurs d'écoulement pharmaceutiquement acceptables.

22. Formulation selon la revendication 21, dans laquelle
(a) chacun desdits un ou plusieurs diluants pharmaceutiquement acceptables est sélectionné dans le groupe consistant en l'amidon prégélatinisé, le lactose monohydraté, le lactose monohydraté de qualité ordinaire, le mannitol, le phosphate de calcium et la cellulose microcristalline ;
(b) chacun desdits un ou plusieurs liants pharmaceutiquement acceptables est sélectionné dans le groupe consistant en la polyvinylpyrrolidone, l'hydroxypropylméthylcellulose, l'hydroxypropylcellulose et l'amidon ;
(c) chacun desdits un ou plusieurs délitants pharmaceutiquement acceptables est sélectionné dans le groupe consistant en la crosscarmellose sodique, l'amidon-glycolate de sodium, la gospovidone et l'amidon ;
(d) chacun desdits un ou plusieurs tensioactifs pharmaceutiquement acceptables est sélectionné dans le groupe consistant en le laurylsulfate de sodium, le polysorbate et le chlorure de cétylpyridinium ;
(e) chacun desdits un ou plusieurs lubrifiants pharmaceutiquement acceptables est sélectionné dans le groupe consistant en le stéarate de magnésium, le stéaryl fumarate de sodium, le béhénate de glycéryle et l'acide stéarique ; ou
(f) chacun desdits un ou plusieurs améliorateurs d'écoulement pharmaceutiquement acceptables est sélectionné dans le groupe consistant en le dioxyde de silicium colloïdal et le talc.

23. Formulation selon la revendication 4, dans laquelle ladite formulation est une solution, et dans laquelle
a) ledit support pharmaceutiquement acceptable comprend un ou plusieurs composés polyoxyhydrocarbyle, un ou plusieurs tensioactifs et un ou plusieurs tampons ;
(b) le composé polyoxyhydrocarbyle est sélectionné dans le groupe consistant en les glucides hydrosolubles, les dérivés de glucides hydrosolubles, les polypeptides hydrosolubles, les polymères hydrosolubles, les polymères oxyalkylène mixtes hydrosolubles, les formes polymères de l'éthylène glycol, et les combinaisons de ceux-ci ;
(c) le tensioactif est sélectionné dans le groupe consistant en les tensioactifs non-ioniques pharmaceutiquement acceptables et les tensioactifs anioniques pharmaceutiquement acceptables ; ou
(d) le tampon est sélectionné dans le groupe consistant en les tampons à base d'acétate, de citrate, d'acide phosphorique, d'ascorbate, d'acide chlorhydrique, de Tris-HCl, de phosphate de sodium, de carbonate de sodium, d'hydroxyde de sodium, de glutamate, de glycine et de Tris-base.

24. Formulation selon la revendication 4, dans laquelle ladite formulation est une suspension aqueuse et dans laquelle
(a) le support pharmaceutiquement acceptable comprend un agent de mise en suspension et un tensioactif ;
(b) l'agent de mise en suspension est sélectionné dans le groupe consistant en la carboxyméthylcellulose, l'hydroxypropylméthylcellulose, la povidone et l'amidon ; ou
(c) le tensioactif est sélectionné dans le groupe consistant en les tensioactifs non-ioniques pharmaceutiquement acceptables et les tensioactifs anioniques pharmaceutiquement acceptables.

25. Formulation selon la revendication 3, dans laquelle
(a) le support pharmaceutiquement acceptable comprend un ou plusieurs tensioactifs pharmaceutiquement acceptables et une ou plusieurs huiles pharmaceutiquement acceptables ;
(b) le tensioactif pharmaceutiquement acceptable comprend un copolymère de l'oxyde d'éthylène et ladite huile pharmaceutiquement acceptable comprend l'huile de sésame ;
(c) l'indolidone substituée ionisable est présente à une concentration sélectionnée dans la gamme allant d'environ 50 mg/g à environ 750 mg/g ;
(d) l'indolidone substituée ionisable est présente à une concentration sélectionnée dans la gamme allant d'environ 50 mg/g à environ 500 mg/g, ou
(e) l'indolidone substituée ionisable est présente à une concentration sélectionnée dans la gamme allant d'environ 50 mg/g à environ 200 mg/g.

26. Formulation selon l'une quelconque des revendications 1-25 formulée dans une gélule de gélatine dure ou dans une gélule de gélatine molle.

27. Utilisation d'une formulation selon l'une quelconque des revendications 1-26 pour la fabrication d'un médicament.

28. Utilisation selon la revendication 27, dans laquelle le médicament est utile dans la prévention ou le traitement d'un trouble lié à une protéine kinase, le trouble lié à une protéine kinase étant un trouble sélectionné dans le groupe consistant en : (a) un cancer qui est sélectionné dans le groupe consistant en le carcinome à cellules squameuses, un sarcome, le sarcome de Kaposi, l'astrocytome, le glioblastome, le cancer du poumon, le cancer de la vessie, le cancer colo-rectal, le cancer gastro-intestinal, le cancer de la tête et du cou, le mélanome, le cancer de l'ovaire, le cancer de la prostate, le cancer du sein, le cancer du poumon à petites cellules et le gliome ; (b) le diabète ; (c) un trouble d'hyperprolifération ; (d) la maladie de Hippel-Lindau ; (e) la resténose ; (f) la fibrose ; (g) le psoriasis ; (h) l'ostéoarthrite ; (i) l'arthrite rhumatoïde ; (j) un trouble inflammatoire ; (k) l'angiogenèse ; (l) un trouble immunologique sélectionné dans le groupe consistant en une maladie auto-immune et le sida ; et (m) un trouble cardio-vasculaire.

29. Utilisation selon la revendication 27 ou 28, dans laquelle l'indolidone substituée ionisable est présente dans le médicament
(a) à une concentration dans la gamme allant d'environ 50 mg/g à environ 750 mg/g ;
(b) à une concentration dans la gamme allant d'environ 50 mg/g à environ 500 mg/g ; ou
(c) à une concentration dans la gamme allant d'environ 50 mg/g à environ 250 mg/g.

30. Procédé de préparation d'une formulation comprenant
(a) l'ajout à une solution saline, formée in situ par mélange d'un équivalent molaire d'une solution basique ou d'une solution acide avec une indolidone substituée ionisable, dans laquelle ladite indolidone substituée ionisable est l'acide 3-[2,4-diméthyl-5-(2-oxo-1,2-dihydro-indol-3-ylidèneméthyl)-1H-pyrrol-3-yl]-propionique o u un sel pharmaceutiquement acceptable ou un dérivé de celle-ci ; et
(b) le mélangeage de la solution de l'indolidone substituée ionisée avec un ou plusieurs composés polyoxyhydrocarbyle et/ou un ou plusieurs tampons.

31. Procédé de préparation d'une formulation pour administration orale, comprenant le mélangeage
(a) d'une indolidone substituée ionisable, dans laquelle ladite indolidone substituée ionisable est l'acide 3-[2,4-diméthyl-5-(2-oxo-1,2-dihydro-indol-3-ylidèneméthyl)-1H-pyrrol-3-yl]-propionique ou un sel pharmaceutiquement acceptable ou un dérivé de celle-ci ;
(b) un ou plusieurs tensioactifs pharmaceutiquement acceptables ; et
(c) une ou plusieurs huiles pharmaceutiquement acceptables.
